(19) Europäisches Patentamt / European Patent Office / Office européen des brevets

(11) EP 2 109 784 B2

(12) **NEW EUROPEAN PATENT SPECIFICATION**
After opposition procedure

(45) Date of publication and mention of the opposition decision:
**05.10.2016 Bulletin 2016/40**

(45) Mention of the grant of the patent:
**26.10.2011 Bulletin 2011/43**

(21) Application number: **08714067.9**

(22) Date of filing: **29.01.2008**

(51) Int Cl.:
***G02B 1/04*** *(2006.01)*

(86) International application number:
**PCT/US2008/052249**

(87) International publication number:
**WO 2008/094876 (07.08.2008 Gazette 2008/32)**

# (54) ANTIMICROBIAL MEDICAL DEVICES INCLUDING SILVER NANOPARTICLES

ANTIMIKROBIELLE MEDIZINISCHE VORRICHTUNGEN MIT SILBERNANOPARTIKELN

DISPOSITIFS MEDICAUX ANTIMICROBIENS COMPORTANT DES NANOPARTICULES D'ARGENT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **31.01.2007 US 887429 P**

(43) Date of publication of application:
**21.10.2009 Bulletin 2009/43**

(73) Proprietor: **Novartis AG**
**4056 Basel (CH)**

(72) Inventors:
- **QIU, Yongxing**
  **Duluth, Georgia 30097 (US)**
- **QIAN, Xinming**
  **Alpharetta, Georgia 30005 (US)**

(74) Representative: **Bohest AG**
**Holbeinstrasse 36-38**
**4051 Basel (CH)**

(56) References cited:
**EP-A- 1 050 314**
**WO-A-02/062402**
**WO-A-2004/047879**
**WO-A2-2005/047879**
**US-A1- 2005 013 842**
**US-A1- 2005 013 842**
**US-A1- 2005 037 057**
**US-A1- 2005 220 882**
**US-A1- 2007 003 603**

- **LI L. ET AL: 'High chemical reactivity of silver nanoparticles toward hydrochloric acid' JOURNAL OF COLLOID AND INTERFACE SCIENCE vol. 303, 2006, pages 415 - 418**
- **ZHANG Z. ET AL: 'PVP Protective Mechanism of Ultrafine Silver Powder Synthesized by Chemical Reduction Processes' JOURNAL OF SOLID STATE CHEMISTRY vol. 121, 1996, pages 105 - 110**
- **LIDE D.R. PH. D. ET AL: 'CRC Handbook of Chemistry and Physics', vol. 78, 1997, CRC PRESS, BOCA RATON - NEW YORK page 8-108**



Printed by Jouve, 75001 PARIS (FR)

## Description

The present invention generally relates to methods for making an antimicrobial contact lens having silver particles distributed therein, and to an antimicrobial contact lens made by said method.

BACKGROUND

Contact lenses are often exposed to one or more microorganisms during wear, storage and handling. They can provide surfaces onto which the microorganisms can adhere and then proliferate to form a colony. Microbial adherence to and colonization of contact lenses may enable microorganisms to proliferate and to be retained on the ocular surface for prolonged periods and thereby may cause infection or other deleterious effects on the ocular health of the eye in which the lens is used. Therefore, it is desirous to make various efforts to minimize and/or eliminate the potential for microorganism adhesion to and colonization of contact lenses.

Many attempts have been made to develop antimicrobial contact lenses, such as, for example, Chalkley et al.'s publication in Am. J. Ophthalmology 1966, 61:866-869 (contact lenses with germicidal agents incorporated therein); U.S. Pat. No. 4,472,327 (contact lenses with antimicrobial agents which may be added to the monomer before polymerization and locked into the polymeric structure of the lenses); U.S. Pat. Nos. 5,358,688 and 5,536,861 and European patent application EP0604369 (contact lenses containing quaternary ammonium group containing organosilicone polymers); European patent application EP0947856A2 (contact lenses containing a quaternary phosphonium group-containing polymer); U.S. Pat. No. 5,515,117 (contact lenses comprising polymeric materials and antimicrobial compounds); U.S. Pat. No. 5,213,801 (contact lenses including an antimicrobial ceramics containing at least one metal selected from Ag, Cu and Zn); U.S. Pat. No. 5,328,954 (contact lenses with coatings composed of a wide variety of antimicrobial agents. In spite of the forgoing efforts, there is no commercially viable contact lenses, especially extended-wear contact lenses, which exhibit antimicrobial activities over a long period of time. WO2002/062402 discloses an optically clear antimicrobial lens, containing greater than 0.01 weight percent activated silver as well as a method for the manufacture of such lens.

A commonly owned co-pending U.S. patent application publication No. 2005/0013842A1 discloses that silver nanoparticles (Ag-nanoparticles) can be incorporated in extended-wear contact lenses to impart to the contact lenses an effective antimicrobial capability over a long period of time. US 2007/003603 discloses a method for making antimicrobial silver compositions comprising silver nanoparticles formed in situ. Said compositions comprise at least one stabilizing agent, one or more silver compounds, at least one reducing agent and a solvent. Although Ag-nanoparticles can be incorporated into contact lenses to impart antimicrobial properties, there are still some issues associated with silver. For example, incorporation of Agnanoparticles can impart to contact lenses undesirable yellowish color. Further, incomplete conversion or reduction of silver ions to silver particles that results in a so-called "staging effect". The remaining silver ions may be slowly converted to silver particles during the storage of cured mold assemblies (cured lenses in molds). Since the cured mold assemblies may be stored ("staged") for different period of time before being further processed through IPA extraction and other steps, the final silver concentration of the lens may vary depending on storage time. As such, the "staging effect" compromises the reproducibility.

Therefore, there is still a need for the development of methods for making anti-microbial contact lenses with silver particles distributed therein. Such methods should be free of at least some issues discussed above and associated with Ag-nanoparticles.

SUMMARY OF THE INVENTION

The invention, in one aspect, provides a method for making an antimicrobial contact lens, even more preferably an antimicrobial extended wear lens. The method comprises the steps of: introducing an amount of a polymerizable dispersion in a mold for making a contact lens; and polymerizing the polymerizable dispersion in the mold to form the antimicrobial contact lens characterized in that the polymerizable dispersion is prepared as follows: obtaining a polymerizable dispersion comprising *in-situ* formed Ag-nanoparticles and a silicone-containing monomer or macromer or prepolymer; treating the Ag-nanoparticles-containing polymerizable dispersion with chloride.

DETAILED DESCRIPTION OF THE PREFERRED EMBODIMENTS

Unless defined otherwise, all technical and scientific terms used herein have the same meaning as commonly understood by one of ordinary skill in the art to which this invention belongs. Generally, the nomenclature used herein and the laboratory procedures are well known and commonly employed in the art. Conventional methods are used for these procedures, such as those provided in the art and various general references. Where a term is provided in the singular, the inventors also contemplate the plural of that term. The nomenclature used herein and the laboratory procedures described below are those well known and commonly employed in the art. As employed throughout the disclosure, the following terms, unless otherwise indicated, shall be understood to have the following meanings.

A "medical device", as used herein, refers to a device or a part thereof having one or more surfaces that contact tissue, blood, or other bodily fluids of patients in the course of their operation or utility. Exemplary medical devices include: (1) extracorporeal devices for use in surgery such as blood oxygenators, blood pumps, blood sensors, tubing used to carry blood and the like which contact blood which is then returned to the patient; (2) prostheses implanted in a human or animal body such as vascular grafts, stents, pacemaker leads, heart valves, and the like that are implanted in blood vessels or in the heart; (3) devices for temporary intravascular use such as catheters, guide wires, and the like which are placed into blood vessels or the heart for purposes of monitoring or repair; (4) artificial tissues such as artificial skin for burn patients; (5) dentifices, dental moldings; (6) ophthalmic devices; and (7) cases or containers for storing ophthalmic devices or ophthalmic solutions.

An "ophthalmic device", as used herein, refers to a contact lens (hard or soft), an intraocular lens, a corneal onlay, other ophthalmic devices (e.g., stents, glaucoma shunt, or the like) used on or about the eye or ocular vicinity.

"Contact Lens" refers to a structure that can be placed on or within a wearer's eye. A contact lens can correct, improve, or alter a user's eyesight, but that need not be the case. A contact lens can be of any appropriate material known in the art or later developed, and can be a soft lens, a hard lens, or a hybrid lens. A "silicone hydrogel contact lens" refers to a contact lens comprising a silicone hydrogel material.

The "front or anterior surface" of a contact lens, as used herein, refers to the surface of the lens that faces away from the eye during wear. The anterior surface, which is typically substantially convex, may also be referred to as the front curve of the lens.

The "rear or posterior surface" of a contact lens, as used herein, refers to the surface of the lens that faces towards the eye during wear. The rear surface, which is typically substantially concave, may also be referred to as the base curve of the lens.

"Biocompatible", as used herein, refers to a material or surface of a material, which may be in intimate contact with tissue, blood, or other bodily fluids of a patient for an extended period of time without significantly damaging the ocular environment and without significant user discomfort.

"Ophthalmically compatible", as used herein, refers to a material or surface of a material which may be in intimate contact with the ocular environment for an extended period of time without significantly damaging the ocular environment and without significant user discomfort. Thus, an ophthalmically compatible contact lens will not produce significant corneal swelling, will adequately move on the eye with blinking to promote adequate tear exchange, will not have substantial amounts of protein or lipid adsorption, and will not cause substantial wearer discomfort during the prescribed period of wear.

"Ocular environment", as used herein, refers to ocular fluids (e.g., tear fluid) and ocular tissue (e.g., the cornea) which may come into intimate contact with a contact lens used for vision correction, drug delivery, wound healing, eye color modification, or other ophthalmic applications.

A "hydrogel" or a "hydrogel material" refers to a polymeric material which can absorb at least 10 percent by weight of water when it is fully hydrated.

A "silicone hydrogel" or a "silicone hydrogel material" refers to a silicone-containing hydrogel obtained by copolymerization of a polymerizable composition comprising at least one silicone-containing vinylic monomer or at least one silicone-containing macromer or at least one crosslinkable silicone-containing prepolymer.

"Hydrophilic," as used herein, describes a material or portion thereof that will more readily associate with water than with lipids.

A "monomer" means a low molecular weight compound that can be polymerized actinically or thermally. Low molecular weight typically means average molecular weights less than 700 Daltons. In accordance with the invention, a monomer can be a vinylic monomer or a compound comprising two thiol groups. A compound with two thiol groups can participate in thiol-ene step-growth radical polymerization with a monomer with vinyl group to form a polymer. Step-growth radical polymerization can be used in making contact lenses, as described in a commonly-owned copending US patent application No. 60/869,812 filed Dec. 13, 2006 (entitled "PRODUCTION OF OPHTHALMIC DEVICES BASED ON PHOTO-INDUCED STEP GROWTH POLYMERIZATION".

A "vinylic monomer", as used herein, refers to a low molecular weight compound that has an ethylenically unsaturated group and can be polymerized actinically or thermally. Low molecular weight typically means average molecular weights less than 700 Daltons.

The term "olefinically unsaturated group" or "ethylenticaly unsaturated group" is employed herein in a broad sense and is intended to encompass any groups containing at least one >C=C< group. Exemplary ethylenically unsaturated groups include without limitation acryloyl, methacryloyl, allyl, vinyl, styrenyl, or other C=C containing groups.

As used herein, "actinically" in reference to curing or polymerizing of a polymerizable composition or material means that the curing (e.g., crosslinked and/or polymerized) is performed by actinic irradiation, such as, for example, UV irradiation, ionized radiation (e.g. gamma ray or X-ray irradiation), microwave irradiation, and the like. Thermal curing

or actinic curing methods are well-known to a person skilled in the art.

The term "fluid" as used herein indicates that a material is capable of flowing like a liquid.

A "hydrophilic monomer" refers to a monomer which can be polymerized actinically or thermally to form a polymer that is water-soluble or can absorb at least 10 percent by weight water.

A "hydrophobic monomer", as used herein, refers to a vinylic monomer which is polymerized actinically or thermally to form a polymer that is insoluble in water and can absorb less than 10 percent by weight water.

A "macromer" refers to a medium and high molecular weight compound which can be polymerized and/or crosslinked actinically or thermally. Medium and high molecular weight typically means average molecular weights greater than 700 Daltons. In accordance with the invention, a macromer comprises one or more ethylenically unsaturated groups and/or one or more thiol groups, which can participate in free radical chain growth polymerization or thiol-ene step-growth radical polymerization. Preferably, a macromer contains ethylenically unsaturated groups and can be polymerized actinically or thermally.

A "prepolymer" refers to a starting polymer which contains crosslinkable groups and can be cured (e.g., crosslinked and/or polymerized) actinically or thermally to obtain a crosslinked and/or polymerized polymer having a molecular weight much higher than the starting polymer. In accordance with the invention, a prepolymer comprises one or more ethylenically unsaturated groups and/or one or more thiol groups, which can participate in free radical chain growth polymerization or thiol-ene step-growth radical polymerization.

A "silicone-containing prepolymer" refers to a prepolymer which contains silicone and can be crosslinked upon actinic radiation or thermally to obtain a crosslinked polymer having a molecular weight much higher than the starting polymer.

"Molecular weight" of a polymeric material (including monomeric or macromeric materials), as used herein, refers to the number-average molecular weight unless otherwise specifically noted or unless testing conditions indicate otherwise.

"Polymer" means a material formed by polymerizing one or more monomers.

A "photoinitiator" refers to a chemical that initiates radical crosslinking/polymerizing reaction by the use of light. Suitable photoinitiators include, without limitation, benzoin methyl ether, diethoxyacetophenone, a benzoylphosphine oxide, 1-hydroxycyclohexyl phenyl ketone, Darocure® types, and Irgacure® types, preferably Darocure® 1173, and Irgacure® 2959.

A "thermal initiator" refers to a chemical that initiates radical crosslinking/polymerizing reaction by the use of heat energy. Examples of suitable thermal initiators include, but are not limited to, 2,2'-azobis (2,4-dimethylpentanenitrile), 2,2'-azobis (2-methylpropanenitrile), 2,2'-azobis (2-methylbutanenitrile), peroxides such as benzoyl peroxide, and the like. Preferably, the thermal initiator is 2,2'-azobis(isobutyronitrile) (AIBN).

An "interpenetrating polymer network (IPN)" as used herein refers broadly to an intimate network of two or more polymers at least one of which is either synthesized and/or crosslinked in the presence of the other(s). Techniques for preparing IPN are known to one skilled in the art. For a general procedure, see U.S. Patent Nos. 4,536,554, 4,983,702, 5,087,392, and 5,656,210. The polymerization is generally carried out at temperatures ranging from about room temperature to about 145°C.

A "spatial limitation of actinic radiation" refers to an act or process in which energy radiation in the form of rays is directed by, for example, a mask or screen or combinations thereof, to impinge, in a spatially restricted manner, onto an area having a well defined peripheral boundary. For example, a spatial limitation of UV radiation can be achieved by using a mask or screen that has a transparent or open region (unmasked region) surrounded by a UV impermeable region (masked region), as schematically illustrated in Figs 1-9 of U.S. Patent No. 6,627,124. The unmasked region has a well defined peripheral boundary with the unmasked region. The energy used for the crosslinking is radiation energy, especially UV radiation, gamma radiation, electron radiation or thermal radiation, the radiation energy preferably being in the form of a substantially parallel beam in order on the one hand to achieve good restriction and on the other hand efficient use of the energy.

"Visibility tinting" in reference to a lens means dying (or coloring) of a lens to enable the user to easily locate a lens in a clear solution within a lens storage, disinfecting or cleaning container. It is well known in the art that a dye and/or a pigment can be used in visibility tinting a lens.

"Dye" means a substance that is soluble in a solvent and that is used to impart color. Dyes are typically translucent and absorb but do not scatter light. Any suitable biocompatible dye can be used in the present invention.

A "Pigment" means a powdered substance that is suspended in a liquid in which it is insoluble. A pigment can be a fluorescent pigment, phosphorescent pigment, pearlescent pigment, or conventional pigment. While any suitable pigment may be employed, it is presently preferred that the pigment be heat resistant, non-toxic and insoluble in aqueous solutions.

"Surface modification", as used herein, means that an article has been treated in a surface treatment process (or a surface modification process), in which, by means of contact with a vapor or liquid, and/or by means of application of an energy source (1) a coating is applied to the surface of an article, (2) chemical species are adsorbed onto the

surface of an article, (3) the chemical nature (e.g., electrostatic charge) of chemical groups on the surface of an article are altered, or (4) the surface properties of an article are otherwise modified. Exemplary surface treatment processes include, but are not limited to, a surface treatment by energy (e.g., a plasma, a static electrical charge, irradiation, or other energy source), chemical treatments, the grafting of hydrophilic monomers or macromers onto the surface of an article, and layer-by-layer deposition of polymeric materials. A preferred class of surface treatment processes are plasma processes, in which an ionized gas is applied to the surface of an article. Plasma gases and processing conditions are described more fully in U.S. Pat. Nos. 4,312,575 and 4,632,844. The plasma gas is preferably a mixture of lower alkanes and nitrogen, oxygen or an inert gas.

"LbL coating", as used herein, refers to a coating that is not covalently attached to a contact lens or a mold half and is obtained through a layer-by-layer ("LbL") deposition of polyionic (or charged) and/or non-charged materials on the lens or mold half. An LbL coating can be composed of one or more layers, preferably one or more bilayers.

As used herein, a "polyionic material" refers to a polymeric material that has a plurality of charged groups or ionizable groups, such as polyelectrolytes, or the likes. Polyionic materials include both polycationic (having positive charges) and polyanionic (having negative charges) materials.

The term "bilayer" is employed herein in a broad sense and is intended to encompass: a coating structure formed on a contact lens or a mold half by alternatively applying, in no particular order, one layer of a first polyionic material (or charged material) and subsequently one layer of a second polyionic material (or charged material) having charges opposite of the charges of the first polyionic material (or the charged material); or a coating structure formed on a contact lens or mold half by alternatively applying, in no particular order, one layer of a first charged polymeric material and one layer of a non-charged polymeric material or a second charged polymeric material. It should be understood that the layers of the first and second coating materials (described above) may be intertwined with each other in the bilayer.

Formation of an LbL coating on a contact lens or mold half may be accomplished in a number of ways, for example, as described in US Patent Ser. No. 6,451,871, 6,719,929, 6,793,973, 6,811,805, 6,896,926.

An "innermost layer", as used herein, refers to the first layer of an LbL coating, which is applied onto the surface of a contact lens or mold half.

A "capping layer" or "outmost layer", as used herein, refers to the last layer or the sole layer of an LbL coating which is applied onto a contact lens or mold half.

An "average contact angle " refers to a water contact angle (advancing angle measured by Wilhelmy Plate method), which is obtained by averaging measurements of at least 3 individual contact lenses.

As used herein, "increased surface hydrophilicity" or "increased hydrophilicity" in reference to a coated contact lens means that the coated contact lens has a reduced averaged contact angle relative to an uncoated contact lens, wherein both coated and uncoated contact lens are made of the same core material.

An "antimicrobial medical device", as used herein, refers to a medical device that exhibit at least a 5-fold reduction (≥80% inhibition), preferably at least a 1-log reduction (≥90% inhibition), more preferably at least a 2-log reduction (≥99% inhibition), of viable microorganisms.

An "antimicrobial agent", as used herein, refers to a chemical that is capable of decreasing or eliminating or inhibiting the growth of microorganisms such as that term is known in the art.

"Ag-nanoparticles" refer to particles which is made essentially of silver metal and have a size of about 1 micrometer or less. Silver in the nanoparticles can be present in one or more of its oxidation states, such as $Ag^0$, $Ag^{1+}$, and $Ag^{2+}$. It is understood that Ag-nanoparticles may undergo aggregation in a fluid composition and the apparent size of Ag-nanoparticles may be several micrometers when analyzed by particle size analyzer without turning on the ultrasonication function of the particle size analyzer (e.g., particle size analyzer Horiba LA-920).

"*in-situ*" formation of Ag-nanoparticles refers to a process in which Ag-nanoparticles are formed directly in a polymerizable fluid composition for making contact lenses. The formation of Ag-nanoparticles can be confirmed by UV spectroscopy with absorption peaks around a wavelength of about 460 nm or smaller, a characteristics of Ag-nanoparticles.

"Chloride-treated Ag-nanoparticles" refer to Ag-nanoparticles obtained according to a process in which after Ag-nanoparticles are formed in a dispersion chloride is added in the dispersion containing the formed Ag-nanoparticles therein so as to reduce substantially the characteristic yellowish color of untreated Ag-nanoparticles.

"Lyophilizing" refers to a freeze-drying process in which the solvent is removed substantially.

"Staging effect" in reference to Ag-nano-particles is intended to describe that because of incomplete conversion or reduction of silver ions to silver particles during in-situ preparation of Ag-nanoparticles, the remaining silver ions may be slowly converted to silver particles during the storage of cured mold assemblies (cured lenses in molds and lens processing (extraction, hydration, other steps) and the final silver concentration (i.e., reproducibility) of the lens may vary depending on storage time and lens processing conditions.

"Stabilized Ag-nanoparticles" refer to Ag-nanoparticles which are formed in the presence of a stabilizer and are stabilized by the stabilizer. Stabilized Ag-nanoparticles can be either positively charged or negatively charged or

neutral, largely depending on a material (or so-called stabilizer) which is present in a solution for preparing Ag-nanoparticles and can stabilize the resultant Ag-nanoparticles. A stabilizer can be any known suitable material. Exemplary stabilizers include, without limitation, positively charged polyionic materials, negatively charged polyionic materials, polymers, surfactants, salicylic acid, alcohols and the like.

The "oxygen transmissibility" of a lens, as used herein, is the rate at which oxygen will pass through a specific ophthalmic lens. Oxygen transmissibility, Dk/t, is conventionally expressed in units of barrers/mm, where t is the average thickness of the material [in units of mm] over the area being measured and "barrer/mm" is defined as:

$$[(cm^3 \text{ oxygen}) / (cm^2)(sec)(mm^2 \text{ Hg})] \times 10^{-9}$$

The intrinsic "oxygen permeability", Dk, of a lens material does not depend on lens thickness. Intrinsic oxygen permeability is the rate at which oxygen will pass through a material. Oxygen permeability is conventionally expressed in units of barrers, where "barrer" is defined as:

$$[(cm^3 \text{ oxygen})(mm) / (cm^2)(sec)(mm^2 \text{ Hg})] \times 10^{-10}$$

These are the units commonly used in the art. Thus, in order to be consistent with the use in the art, the unit "barrer" will have the meanings as defined above. For example, a lens having a Dk of 90 barrers ("oxygen permeability barrers") and a thickness of 90 microns (0.090 mm) would have a Dk/t of 100 barrers/mm (oxygen transmissibility barrers/mm). In accordance with the invention, a high oxygen permeability in reference to a material or a contact lens characterized by apparent oxygen permeability of at least 40 barrers or larger measured with a sample (film or lens) of 100 microns in thickness according to a coulometric method described in Examples.

The "ion permeability" through a lens correlates with both the Ionoflux Diffusion Coefficient and the Ionoton Ion Permeability Coefficient.

The Ionoflux Diffusion Coefficient, D, is determined by applying Fick's law as follows:

$$D = - n' / (A \times dc/dx)$$

where

n' = rate of ion transport [mol/min]
A = area of lens exposed [$mm^2$]
D = Ionoflux Diffusion Coefficient [$mm^2$/min]
dc = concentration difference [mol/L]
dx = thickness of lens [mm]

The Ionoton Ion Permeability Coefficient, P, is then determined in accordance with the following equation:

$$\ln( 1 - 2C(t)/C(0) ) = -2APt / Vd$$

where:

C(t) = concentration of sodium ions at time t in the receiving cell
C(0) = initial concentration of sodium ions in donor cell
A = membrane area, i.e., lens area exposed to cells
V = volume of cell compartment (3.0 ml)
d = average lens thickness in the area exposed
P = permeability coefficient

An Ionoflux Diffusion Coefficient, D, of greater than about 0.2 x $10^{-3}$ $mm^2$/min is preferred, while greater than about 0.64 x $10^{-3}$ $mm^2$/min is more preferred and greater than about 1.0 x $10^{-3}$ $mm^2$/min is most preferred.

It is known that on-eye movement of the lens is required to ensure good tear exchange, and ultimately, to ensure good corneal health. Ion permeability is one of the predictors of on-eye movement, because the permeability of

ions is believed to be directly proportional to the permeability of water.

The water content of a lens can be measured according to Bulk Technique as disclosed in US 5,760,100. Preferably, the lens has a water content of at least 15% by weight when fully hydrated, based on the total lens weight.

The present invention is generally directed to methods for making an antimicrobial contact lens having silver particles distributed uniformly therein and to an antimicrobial contact lens made therefrom. The present invention is partly based on the discovery that by simply treating, with chloride, a lens formulation containing silver nano-particles prepared *in situ,* one converts the free silver ions to silver chloride and unexpectedly, the color of the formulation can also be changed from yellowish to blue. The present invention is also partly based on the discovery that the process condition of adding chloride is very important in order to avoid aggregation of silver particles. By having a step of chloride treatment, the staging effect of Agnanoparticles is minimized and the reproducibility in the final concentration of silver in a lens is controllable. Chloride can be added via hydrochloride or sodium chloride (NaCl). Certain process conditions are important such as the timing of adding chloride, the concentration, whether adding dissolved NaCl or NaCl solid, etc. In addition, the relative concentration of stabilizer to silver is also important in achieving the desired properties such as color and acceptable particle size. The present invention further is partly based on the discovery that an antimicrobial contact lens, which has silver nano-particles distributed uniformly therein, can be produced according to one of cost-effective and efficient processes developed herein. It is believed that silver nano-particles can release, at an extremely slow rate, silver ions which in turn can leach slowly out of a contact lens and therefore decrease or eliminate or inhibit the growth of microorganisms. It is also believed that the chloride treatment may provide more than one silver source (silver nanoparticles and silver chloride) which can increase the silver release. By using a process of the invention, one can incorporate silver particles uniformly in the polymer matrix of the contact lens to impart antimicrobial capability without significantly adverse effects on the desired bulk properties of the contact lens, such as oxygen permeability, ion or water permeability.

The invention, in one aspect, provides a method for making an antimicrobial contact lens, even more preferably an antimicrobial extended wear lens. The method comprises the steps of: introducing an amount of a polymerizable dispersion in a mold for making a contact lens; and polymerizing the polymerizable dispersion in the mold to form the antimicrobial contact lens characterized in that the polymerizable dispersion is prepared as follows: obtaining a polymerizable dispersion comprising *in-situ* formed Ag-nanoparticles and a silicone-containing monomer or macromer or prepolymer; treating the Ag-nanoparticles-containing polymerizable dispersion with chloride.

In one embodiment, *in-situ* formation of Ag-nanoparticles is performed by adding a desired amount of a soluble silver salt into a polymerizable fluid composition comprising a monomer capable of reducing silver cations and a silicone-containing monomer or macromer or prepolymer so as to form a mixture and by mixing thoroughly the mixture for a period of time long enough to reduce at least 20%, preferably at least 50%, more preferably at least 80%, even more preferably at least 95% of the added amount of the silver salt into silver nanoparticles so as to form a polymerizable dispersion.

In another embodiment, *in-situ* formation of Ag-nanoparticles is performed by adding into a polymerizable fluid composition comprising a silicone-containing monomer or macromer or prepolymer at least one biocompatible reducing agent to form a mixture, wherein the amount of the biocompatible reducing agent added in the mixture is sufficient to reduce at least 20%, preferably at least 50%, more preferably at least 80%, even more preferably at least 95% of the silver salt into Ag-nanoparticles; and by mixing thoroughly the mixture for a period of time sufficient to reduce at least 20%, preferably at least 50%, more preferably at least 80%, even more preferably at least 95% of the silver salt into Ag-nanoparticles so as to form a polymerizable dispersion.

In accordance with the present invention, a polymerizable fluid composition can be a solution or a solvent-free liquid or melt at a temperature below 60°C.

Where a polymerizable fluid composition is a solution, it can be prepared by dissolving at least one silicone-containing monomer, macromer or prepolymer and all other desired components in any suitable solvent known to a person skilled in the art. Examples of suitable solvents are water, alcohols, such as lower alkanols, for example ethanol or methanol, and furthermore carboxylic acid amides, such as dimethylformamide, dipolar aprotic solvents, such as dimethyl sulfoxide or methyl ethyl ketone, ketones, for example acetone or cyclohexanone, hydrocarbons, for example toluene, ethers, for example THF, dimethoxyethane or dioxane, and halogenated hydrocarbons, for example trichloroethane, and also mixtures of suitable solvents, for example mixtures of water with an alcohol, for example a water/ethanol or a water/methanol mixture.

Any known suitable silicone-containing monomers can be used in the present invention. In accordance with the invention, a monomer can be a silicone-containing vinylic monomer or a monomer with two thiol groups. Examples of silicone-containing monomers include, without limitation, methacryloxyalkylsiloxanes, 3-methacryloxy propylpentamethyldisiloxane, bis(methacryloxypropyl)tetramethyl-disiloxane, monomethacrylated polydimethylsiloxane, mercapto-terminated polydimethylsiloxane, N-[tris(trimethylsiloxy)silylpropyl]acrylamide, N-[tris(trimethylsiloxy)silylpropyl]methacrylamide, tris(pentamethyldisiloxyanyl)-3-methacrylatopropylsilane (T2), and tristrimethylsilyloxysilylpropyl methacrylate (TRIS). A preferred siloxane-containing monomer is TRIS, which is referred to 3-methacryloxypropyltris(trimethylsiloxy) silane, and represented by CAS No. 17096-07-0. The term "TRIS" also includes dimers of 3-methacryloxypropyltris(trimethylsiloxy) silane. The silicone-containing monomer can also comprise one or more hydroxyl and/or amino groups.

Where the polymerization of the polymerizable dispersion is carried out based on thiol-ene step-growth radical polymerization, the silicone-containing monomer preferably comprises two thiol groups or one ene-containing group defined by any one of formula (I) - (III)

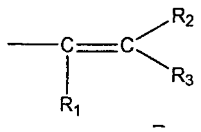

(I)

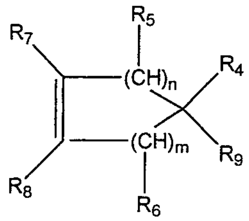

(II)

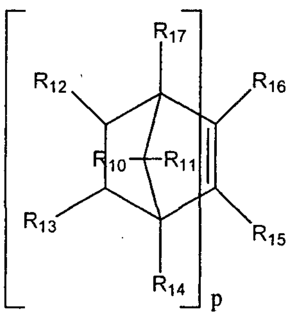

(III)

in which $R_1$ is hydrogen, or $C_1$-$C_{10}$ alkyl; $R_2$ and $R_3$ independent of each other are hydrogen, $C_1$-$C_{10}$ alkene divalent radical, $C_1$-$C_{10}$ alkyl, or $-(R_{18})_a-(X_1)_b-R_{19}$ in which $R_{18}$ is $C_1$-$C_{10}$ alkene divalent radical, $X_1$ is an ether linkage (-O-), a urethane linkage (-N), a urea linkage, an ester linkage, an amid linkage, or carbonyl, $R_{19}$ is hydrogen, a single bond, amino group, carboxylic group, hydroxyl group, carbonyl group, $C_1$-$C_{12}$ aminoalkyl group, $C_1$-$C_{18}$ alkylaminoalkyl group, $C_1$-$C_{18}$ carboxyalkyl group, $C_1$-$C_{18}$ hydroxyalkyl group, $C_1$-$C_{18}$ alkylalkoxy group, $C_1$-$C_{12}$ aminoalkoxy group, $C_1$-$C_{18}$ alkylaminoalkoxy group, $C_1$-$C_{18}$ carboxyalkoxy group, or $C_1$-$C_{18}$ hydroxyalkoxy group, a and b independent of each other is zero or 1, provided that only one of $R_2$ and $R_3$ is a divalent radical; $R_4$ - $R_9$, independent of each other, are hydrogen, $C_1$-$C_{10}$ alkene divalent radical, $C_1$-$C_{10}$ alkyl, or $-(R_{18})_a-(X_1)_b-R_{19}$, optionally $R_4$ and $R_9$ are linked through an alkene divalent radical to form a cyclic ring, provided that at least one of $R_4$-$R_9$ are divalent radicals; n and m independent of each other are integer number from 0 to 9, provided that the sum of n and m is an integer number from 2 to 9; $R_{10}$ - $R_{17}$, independent of each other, are hydrogen, $C_1$-$C_{10}$ alkene divalent radical, $C_1$-$C_{10}$ alkyl, or $-(R_{18})_a-(X_1)_b-R_{19}$, p is an integer number from 1 to 3, provided that only one or two of $R_{10}$-$R_{17}$ are divalent radicals.

Any know suitable silicone-containing macromers can be used in the invention. In accordance with the invention, a macromer comprises one or more ethylenically unsaturated groups and/or at least two thiol groups, which can participate in free radical chain growth polymerization or thiol-ene step-growth radical polymerization. Preferably, a silicone-containing macromer is a siloxane-containing macromer. Any suitable siloxane-containing macromer with ethylenically unsaturated group(s) can be used to produce a silicone hydrogel material. A particularly preferred siloxane-containing macromer is selected from the group consisting of Macromer A, Macromer B, Macromer C, and Macromer D described in US 5,760,100. Macromers that contain two or more polymerizable groups (vinylic groups) can also serve as cross linkers. Di and triblock macromers consisting of polydimethylsiloxane and polyakyleneoxides could also be of utility. Such macromers could be mono or difunctionalized with acrylate, methacrylate or vinyl groups. For example one might use methacrylate end capped polyethyleneoxide-block-polydimethylsiloxane-block-polyethyleneoxide to enhance oxygen permeability.

Where the polymerization of the polymerizable dispersion is carried out based on thiol-ene step-growth radical

polymerization, the silicone-containing macromer preferably comprises at least two thiol groups or one or more ene-containing groups defined by any one of formula (I) - (III) above.

In accordance with the invention, a prepolymer comprises one or more ethylenically unsaturated groups and/or at least two thiol groups, which can participate in free radical chain growth polymerization or thiol-ene step-growth radical polymerization. Examples of silicone-containing prepolymers include without limitation those disclosed in US Patent Application Publication No. US 2001-0037001 A1, US Patent No. 6,039,913, and a co-pending US patent application serial No. 60/869,812 filed Dec. 13, 2006 (entitled "PRODUCTION OF OPHTHALMIC DEVICES BASED ON PHOTO-INDUCED STEP GROWTH POLYMERIZATION". Preferably, the prepolymers used in the invention are previously purified in a manner known per se, for example by precipitation with organic solvents, such as acetone, filtration and washing, extraction in a suitable solvent, dialysis or ultrafiltration, ultrafiltration being especially preferred. By means of that purification process the prepolymers can be obtained in extremely pure form, for example in the form of concentrated aqueous solutions that are free, or at least substantially free, from reaction products, such as salts, and from starting materials, such as, for example, non-polymeric constituents. The preferred purification process for the prepolymers used in the process according to the invention, ultrafiltration, can be carried out in a manner known per se. It is possible for the ultrafiltration to be carried out repeatedly, for example from two to ten times. Alternatively, the ultrafiltration can be carried out continuously until the selected degree of purity is attained. The selected degree of purity can in principle be as high as desired. A suitable measure for the degree of purity is, for example, the concentration of dissolved salts obtained as by-products, which can be determined simply in known manner.

Where the polymerization of the polymerizable dispersion is carried out based on thiol-ene step-growth radical polymerization, the silicone-containing prepolymer preferably comprises at least two thiol groups or one or more ene-containing groups defined by any one of formula (I) - (III) above.

In accordance with the present invention, a polymerizable fluid composition can also comprise a hydrophilic monomer. Nearly any hydrophilic monomer that can act as a plasticizer can be used in the fluid composition of the invention. Among the preferred hydrophilic vinylic monomers are N,N-dimethylacrylamide (DMA), 2-hydroxyethylmethacrylate (HEMA), hydroxyethyl acrylate, hydroxypropyl acrylate, hydroxypropyl methacrylate (HPMA), trimethylammonium 2-hydroxy propylmethacrylate hydrochloride, dimethylaminoethyl methacrylate (DMAEMA), dimethylaminoethylmethacrylamide, acrylamide, methacrylamide, allyl alcohol, vinylpyridine, glycerol methacrylate, N-(1,1dimethyl-3-oxobutyl)acrylamide, N-vinyl-2-pyrrolidone (NVP), acrylic acid, methacrylic acid, and N,N-dimethyacrylamide (DMA).

A polymerizable fluid composition can also comprises a hydrophobic monomer. By incorporating a certain amount of hydrophobic monomer in a polymerizable fluid composition, the mechanical properties (e.g., modulus of elasticity) of the resultant polymer may be improved.

In a preferred embodiment, a polymerizable fluid composition suitable for making a contact lens will include (a) about 20 to 40 weight percent of a siloxane-containing macromer, (b) about 5 to 30 weight percent of a siloxane-containing monomer, and (c) about 10 to 35 weight percent of a hydrophilic monomer. More preferably, the siloxane-containing monomer is TRIS.

In accordance with the present invention, a polymerizable fluid composition can further comprise various components, such as cross-linking agents, a chain transfer agent, initiator, UV-absorbers, inhibitors, fillers, visibility tinting agents (e.g., dyes, pigments, or mixtures thereof), non-crosslinkable hydrophilic polymers as leacheable wetting agents, and the like, as known to a person skilled in the art.

Cross-linking agents may be used to improve structural integrity and mechanical strength. Examples of cross-linking agents include without limitation allyl(meth)acrylate, lower alkylene glycol di(meth)acrylate, poly lower alkylene glycol di(meth)acrylate, lower alkylene di(meth)acrylate, divinyl ether, divinyl sulfone, di- or trivinylbenzene, trimethylolpropane tri(meth)acrylate, pentaerythritol tetra(meth)acrylate, bisphenol A di(meth)acrylate, methylenebis(meth)acrylamide, triallyl phthalate or diallyl phthalate. A preferred cross-linking agent is ethylene glycol dimethacrylate (EGDMA).

The amount of a cross-linking agent used is expressed in the weight content with respect to the total polymer and is in the range from 0.05 to 20%, in particular in the range from 0.1 to 10 %, and preferably in the range from 0.1 to 2%.

Initiators, for example, selected from materials well known for such use in the polymerization art, may be included in the polymerizable fluid composition in order to promote, and/or increase the rate of, the polymerization reaction. An initiator is a chemical agent capable of initiating polymerization reactions. The initiator can be a photoinitiator or a thermal initiator.

A photoinitiator can initiate free radical polymerization and/or crosslinking by the use of light. Suitable photoinitiators are benzoin methyl ether, diethoxyacetophenone, a benzoylphosphine oxide, 1-hydroxycyclohexyl phenyl ketone and Darocur and Irgacur types, preferably Darocur 1173® and Darocur 2959®. Examples of benzoylphosphine initiators include 2,4,6-trimethylbenzoyldiphenylophosphine oxide; bis-(2,6-dichlorobenzoyl)-4-N-propylphenylphosphine oxide; and bis-(2,6-dichlorobenzoyl)-4-N-butylphenylphosphine oxide. Reactive photoinitiators which can be incorporated, for example, into a macromer or can be used as a special monomer are also suitable. Examples of reactive photoinitiators are those disclosed in EP 632 329, herein incorporated by reference in its entirety. The polymerization can then be triggered off by actinic radiation, for example light, in particular UV light of a suitable wavelength. The spectral requirements

can be controlled accordingly, if appropriate, by addition of suitable photosensitizers

Examples of suitable thermal initiators include, but are not limited to, 2,2'-azobis (2,4-dimethylpentanenitrile), 2,2'-azobis (2-methylpropanenitrile), 2,2'-azobis (2-methylbutanenitrile), peroxides such as benzoyl peroxide, and the like. Preferably, the thermal initiator is azobisisobutyronite (AIBN).

Examples of preferred pigments include any colorant permitted in medical devices and approved by the FDA, such as D&C Blue No. 6, D&C Green No. 6, D&C Violet No. 2, carbazole violet, certain copper complexes, certain chromium oxides, various iron oxides, phthalocyanine green, phthalocyanine blue, titanium dioxides, etc. See Marmiom DM Handbook of U.S. Colorants for a list of colorants that may be used with the present invention. A more preferred embodiment of a pigment include (C.I. is the color index no.), without limitation, for a blue color, phthalocyanine blue (pigment blue 15:3, C.I. 74160), cobalt blue (pigment blue 36, C.I. 77343), Toner cyan BG (Clariant), Permajet blue B2G (Clariant); for a green color, phthalocyanine green (Pigment green 7, C.I. 74260) and chromium sesquioxide; for yellow, red, brown and black colors, various iron oxides; PR122, PY154, for violet, carbazole violet; for black, Monolith black C-K (CIBA Specialty Chemicals).

Any non-crosslinkable hydrophilic polymers can be used in the invention as leachable wetting agents. Exemplary non-crosslinkable hydrophilic polymers include, but are not limited to, polyvinylalcohols (PVAs), polyethylene oxide, polyethylene-polypropylene block copolymers, polyamides, polyimides, polylactone, a homopolymer of a vinyl lactam, a copolymer of at least one vinyl lactam in the presence or in the absence of one or more hydrophilic vinylic comonomers, a homopolymer of acrylamide or methaacrylamide, a copolymer of acrylamide or methacrylamide with one or more hydrophilic vinylic monomers, mixtures thereof.

In accordance with the invention, the vinyl lactam has a structure of formula (IV)

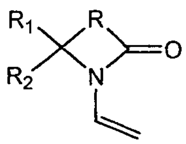

(IV)

wherein R is an alkylene di-radical having from 2 to 8 carbon atoms; $R_1$ is hydrogen, alkyl, aryl, aralkyl or alkaryl, preferably hydrogen or lower alkyl having up to 7 carbon atoms, and, more preferably, up to 4 carbon atoms, such as, for example, methyl, ethyl or propyl; aryl having up to 10 carbon atoms, and also aralkyl or alkaryl having up to 14 carbon atoms; and $R_2$ is hydrogen or lower alkyl having up to 7 carob atoms and, more preferably, up to 4 carbon atoms, such as, for example, methyl, ethyl or propyl.

A non-crosslinkable hydrophilic polymer is present in the a polymerizable fluid composition in an amount sufficient to render a formed silicone hydrogel lens having a wettable and durable coating, for example, in an amount of from about 0.5% to about 10 % by weight, preferably from about 1% to about 8.0 % by weight, and more preferably from about 3% to about 6 % by weight, each based on the entire weight of the composition.

The number-average molecular weight $M_n$ of a non-crosslinkable hydrophilic polymer is at least 40000 daltons, preferably at least 80000 daltons, more preferably at least 100000 daltons, even more preferably at least 250000 daltons.

Examples of hydrophilic polymers include but are not limited to polyvinylalcohol (PVA), polyethylene oxide (i.e., polyethyleneglycol (PEG)), providone, copolymers of vinyl pyrrolidone/dimethylaminoethylmethacrylate, copolymers of vinyl pyrrolidone/vinyl acetate, alkylated polyvinylpyrrolidone, copolymers of vinyl pyrrolidone/dimethylaminoethylmethacrylate, copolymers of vinylpyrrolidone/acrylic acid, poly-N-vinyl-2-piperidone, poly-N-vinyl-2-caprolactam, poly-N-vinyl-3-methyl-2-caprolactam, poly-N-vinyl-3-methyl-2-piperidone, poly-N-vinyl-4-methyl-2-piperidone, poly-N-vinyl-4-methyl-2-caprolactam, poly-N-vinyl-3-ethyl-2-pyrrolidone, and poly-N-vinyl4,5-dimethyl-2-pyrrolidone, polyvinylimidazole, poly-N-N-dimethylacrylamide, polyacrylic acid, poly 2 ethyl oxazoline, heparin polysaccharides, polysaccharides, a polyoxyethylene derivative, mixtures thereof.

A suitable polyoxyethylene derivative is, for example, a n-alkylphenyl polyoxyethylene ether, n-alkyl polyoxyethylene ether (e.g., TRITON®), polyglycol ether surfactant (TERGITOL®), polyoxyethylenesorbitan (e.g., TWEEN®), polyoxyethylated glycol monoether (e.g., BRIJ®, polyoxylethylene 9 lauryl ether, polyoxylethylene 10 ether, polyoxylethylene 10 tridecyl ether), or a block copolymer of ethylene oxide and propylene oxide (e.g. poloxamers or poloxamines).

A class of preferred polyoxyethylene derivatives used in the present invention are polyethylene-polypropylene block copolymers, in particular poloxamers or poloxamines which are available, for example, under the tradename PLURONIC®, PLURONIC-R®, TETRONIC®, TETRONIC-R® or PLURADOT®.

Poloxamers are triblock copolymers with the structure PEO-PPO-PEO (where "PEO" is poly(ethylene oxide) and "PPO" is poly(propylene oxide). A considerable number of poloxamers is known, differing merely in the molecular weight and in the PEO/PPO ratio; Examples are poloxamer 101, 105, 108, 122, 123, 124, 181, 182, 183, 184, 185, 188, 212, 215, 217, 231, 234, 235, 237, 238, 282, 284, 288, 331, 333, 334, 335, 338, 401, 402, 403 and 407. The poloxamers may be used in the process of the invention irrespective of their PEO/PPO ratio; for example, poloxamer 101 having a

PEO/PPO weight ratio of about 10/90 and poloxamer 108 having a PEO/PPO weight ratio of about 80/20 both have been found to be valuable as non-crosslinkable polymer in the aqueous solution according to step a).

The order of polyoxyethylene and polyoxypropylene blocks can be reversed creating block copolymers with the structure PPO-PEO-PPO, which are known as PLURONIC-R® polymers.

Poloxamines are polymers with the structure $(PEO\text{-}PPO)_2\text{-}N\text{-}(CH_2)_2\text{-}N\text{-}(PPO\text{-}PEO)_2$ that are available with different molecular weights and PEO/PPO ratios. Again, the order of polyoxyethylene and polyoxypropylene blocks can be reversed creating block copolymers with the structure $(PPO\text{-}PEO)_2\text{-}N\text{-}(CH_2)_2\text{-}N\text{-}(PEO\text{-}PPO)_2$, which are known as TETRONIC-R® polymers.

Polyoxypropylene-polyoxyethylene block copolymers can also be designed with hydrophilic blocks comprising a random mix of ethylene oxide and propylene oxide repeating units. To maintain the hydrophilic character of the block, ethylene oxide will predominate. Similarly, the hydrophobic block can be a mixture of ethylene oxide and propylene oxide repeating units. Such block copolymers are available under the tradename PLURADOT®.

PVA is a highly biocompatible material used widely in ophthalmic products, especially wetting drops or artificial tears for ocular comfort (e.g., HypoTears™, etc.). Non-crosslinkable PVAs of all kinds, for example those with low, medium or high polyvinyl acetate contents may be employed. The non-crosslinkable polyvinyl alcohols employed in the present invention are known and are commercially available, for example under the brand name Mowiol® from KSE (Kuraray Specialties Europe).

Preferably, a polymerizable fluid composition comprises at least one high molecular weight non-crosslinkable PVA with a $M_n$ of from above 50000 to 100000, preferably from above 50000 to 75000 and at least one low molecular weight non-crosslinkable PVA with a $M_n$ of from 25000 to 50000, preferably from 30000 to 50000.

In case of two or more different non-crosslinkable PVAs, the total amount thereof in the composition is as described before including the preferences given. The weight proportion of the lower molecular weight and higher molecular weight non-crosslinkable PVA may vary within broad ranges, but is, for example, from 1:1 to 5:1, preferably from 1:1 to 4:1, and in particular from 1:1 to 3:1.

A mixture of non-crosslinkable PVAs and polyethyleneglycol (PEG) can be used in the invention. PVA and PEG may have synergy for enhancing surface wettability of a silicone hydrogel contact lens.

It has been found that some classes of monomers can reduce silver ions into silver nano-particles. Examples of such monomers include without limitation acrylamide, methacrylamide, di(lower alkyl)acrylamides, di(lower alkyl)methacrylamides, (lower allyl)acrylamides, (lower allyl)methacrylamides, hydroxyl-substituted (lower alkyl)acrylamides, hydroxyl-substituted (lower alkyl)methacrylamides, and N-vinyl lactams.

Exemplary N-vinyl lactams include without limitation N-vinyl-2-pyrrolidone (NVP), N-vinyl-2-piperidone, N-vinyl-2-caprolactam, N-vinyl-3-methyl-2-pyrrolidone, N-vinyl-3-methyl-2-piperidone, N-vinyl-3-methyl-2-caprolactam, N-vinyl-4-methyl-2-pyrrolidone, N-vinyl-4-methyl-2-caprolactam, N-vinyl-5-methyl-2-pyrrolidone, N-vinyl-5-methyl-2-piperidone, N-vinyl-5,5-dimethyl-2-pyrrolidone, N-vinyl-3,3,5-trimethyl-2-pyrrolidone, N-vinyl-5-methyl-5-ethyl-2-pyrrolidone, N-vinyl-3,4,5-trimethyl-3-ethyl-2-pyrrolidone, N-vinyl-6-methyl-2-piperidone, N-vinyl-6-ethyl-2-piperidone, N-vinyl-3,5-dimethyl-2-piperidone, N-vinyl-4,4-dimethyl-2-piperidone, N-vinyl-7-methyl-2-caprolactam, N-vinyl-7-ethyl-2-caprolactam, N-vinyl-3,5-dimethyl-2-caprolactam, N-vinyl-4,6-dimethyl-2-caprolactam and N-vinyl-3,5,7-trimethyl-2-caprolactam.

A person skilled in the art will know how to determine which monomers are capable of reducing silver ions into silver nano-particles. In a preferred embodiment, a monomer capable of reducing silver ions into nano-particles is N-dimethylacrylamide (DMA) or N-vinyl-2-pyrrolidone (NVP).

Any suitable biocompatible reducing agents can be used in the invention. Examples of biocompatible reducing agents includes without limitation ascorbic acid and biocompatible salts thereof, and biocompatible salts of citrate.

Any known suitable soluble silver salts can be used in the present invention. Preferably, silver nitrate is used.

It has been found that a siloxane-containing macromer having hydrophilic units can stabilize silver nano-particles. A polymerizable dispersion containing Ag-nanoparticles and a siloxane-containing macromer having hydrophilic units can be stable for a relatively long period of time, for example, at least two hours. A stable polymerizable dispersion can provide more flexibility in producing antimicrobial contact lenses in which Ag-nanoparticles are uniformly distributed. It should be understood that the addition of a hydrophilic and/or hydrophobic monomer can also improve the stability of the polymerizable dispersion with Ag-nanoparticles, probably due to synergy among them. For example, a polymerizable dispersion prepared from a lens formulation can be more stable than a dispersion prepared from each individual components of that lens formulation.

In a preferred embodiment of the invention, a polymerizable fluid composition comprises a stabilizer for stabilizing Ag-nanoparticles. A "stabilizer" refers to a material which is present in a solution for preparing the nano-particles and can stabilize the resultant nano-particles. A small amount of a stabilizer present in the polymerizable dispersion can improve greatly the stability of the polymerizable dispersion. In accordance with the present invention, a stabilizer can be a polyanionic material, a polycationic material, or a polyvinylpyrrolidone (PVP) or a copolymer of n-vinylpyrrolidone with one ore more vinylic monomers.

A polycationic material used in the present invention can generally include any material known in the art to

have a plurality of positively charged groups along a polymer chain. For instance, suitable examples of such polycationic materials can include, but are not limited to, poly(allylamine hydrochloride) (PAH), poly(ethyleneimine) (PEI), poly(vinylbenzyltriamethylamine) (PVBT), polyaniline (PAN or PANI) (p-type doped) [or sulphonated polyaniline], polypyrrole (PPY) (p-typed doped), and poly(pyridinium acetylene).

A polyanionic material used in the present invention can generally include any material known in the art to have a plurality of negatively charged groups along a polymer chain. For example, suitable polyanionic materials can include, but are not limited to, polymethacrylic acid (PMA), polyacrylic acid (PAA), poly(thiophene-3-acetic acid) (PTAA), poly(4-styrenesulfonic acid) (PSS), sodium poly(styrene sulfonate) (SPS) and poly(sodium styrene sulfonate) (PSSS).

The foregoing lists are intended to be exemplary, but clearly are not exhaustive. A person skilled in the art, given the disclosure and teaching herein, would be able to select a number of other useful polyionic materials including a synthetic polymer, a biopolymer or a modified biopolymer.

A preferred stabilizer is polyacrylic acid (PAA), poly(ethyleneimine) (PEI), polyvinylpyrrolidone of a molecular weight of up to 1,500,000, a copolymer (of a molecular weight of up to 1,500,000) of vinylpyrrolidone with one or more vinylic monomer, a polyionic material having amino groups and/or sulfur-containing groups or mixture thereof.

Exemplary sulfur-containing groups include, without limitation, thiol, sulfonyl, sulfonic acid, alkyl sulfide, alkyl disulfide, substituted or unsubstituted phenyldisulfide, thiophenyl, thiourea, thioether, thiazolyl, thiazolinyl, and the like.

The amount of a stabilizer in a polymerizable fluid composition is less than 1% percent by weight, preferably less than 0.5% by weight, more preferably less than 0.1 % by weight.

Alternatively, a stabilizer can be added into a polymerizable fluid composition together with soluble silver salt (e.g., a solution of $AgNO_3$ and PAA). The concentration ratio of a stabilizer to silver nano-particles is preferably from 0.1 to 10, more preferably from 0.5 to 5.

It should point out that where a stabilizer is -COOH-containing polymer (e.g., PAA), an amino-containing polycationic polymer, or a sulfur-containing polyionic polymer, the concentration of the stabilizer should be at a level below which silver ions can be reduced into Ag-nanoparticles. If the stabilizer concentration is too high, the reduction of silver ions into Ag-nanoparticles can be extremely slow or almost inhibited.

In accordance with the invention, "treating of the Ag-nanoparticles-containing polymerizable dispersion with chloride" refers to introducing of chloride ions into the polymerizable dispersion.

In one embodiment, treating of the Ag-nanoparticles-containing polymerizable dispersion with chloride can be performed by: (1) adding chloride salt, such as NaCl in solid form, directly into the dispersion; (2) mixing thoroughly the mixture for a period of time long enough to substantially reduce yellowish color of Ag-nanoparticles in the dispersion; and (3) removing remaining solid chloride salt. Such method (by adding solid chloride salt directly in the dispersion) is especially suitable for a dispersion the solvent of which is an organic solvent, a mixture of organic solvents, or a mixture of water and an organic solvent. Its advantage is that such chloride treatment would not change significantly the concentration of the components in the dispersion. Removal of solid chloride salt can be preformed by means of filtration, or any known methods.

In another embodiment, the chloride treatment can be carried out by: (1) adding a concentrated NaCl solution or concentrated hydrochloride into the dispersion and (2) mixing thoroughly the mixture for a period of time long enough to substantially reduce yellowish color of Ag-nanoparticles in the dispersion.

Contact lenses of the invention can be made in a manner known per se from a polymerizable fluid dispersion by a polymerization reaction in molds for making the contact lens with which the expert is familiar. For example, a contact lens may be manufactured, generally, by thoroughly mixing the polymer composition of the present invention, applying an appropriate amount of the mixture to a lens mold cavity, and initiating polymerization. Photoinitiators, such as those commercially available photoinitiators, e.g., DAROCUR® 1173 (a photoinitator available from Ciba-Geigy Corporation), may be added to the polymer composition to aid in initiating polymerization. Polymerization may be initiated actinically or thermally. A preferred method of initiating polymerization is by application of actinic radiation.

Methods of forming mold sections for cast-molding a contact lens are generally well known to those of ordinary skill in the art. The process of the present invention is not limited to any particular method of forming a mold. In fact, any method of forming a mold can be used in the present invention. However, for illustrative purposes, the following discussion has been provided as one embodiment of forming a contact lens mold.

Lens molds for making contact lenses are well known to a person skilled in the art and, for example, are employed in cast molding or spin casting. For example, a mold (for cast molding) generally comprises at least two mold sections (or portions) or mold halves, i.e. first and second mold halves. The first mold half defines a first molding (or optical) surface and the second mold half defines a second molding (or optical) surface. The first and second mold halves are configured to receive each other such that a lens forming cavity is formed between the first molding surface and the second molding surface. The molding surface of a mold half is the cavity-forming surface of the mold and in direct contact with lens-forming material.

Methods of manufacturing mold sections for cast-molding a contact lens are generally well known to those of ordinary skill in the art. The process of the present invention is not limited to any particular method of forming a mold.

In fact, any method of forming a mold can be used in the present invention. The first and second mold halves can be formed through various techniques, such as injection molding or lathing. Examples of suitable processes for forming the mold halves are disclosed in U.S. Patent Nos. 4,444,711 to Schad; 4,460,534 to Boehm et al.; 5,843,346 to Morrill; and 5,894,002 to Boneber et al..

Virtually all materials known in the art for making molds can be used to make molds for preparing ocular lenses. For example, polymeric materials, such as polyethylene, polypropylene, polystyrene, PMMA, cyclic olefin copolymers (e.g., Topas® COC from Ticona GmbH of Frankfurt, Germany and Summit, New Jersey; Zeonex® and Zeonor® from Zeon Chemicals LP, Louisville, KY), or the like can be used. Other materials that allow UV light transmission could be used, such as quartz glass and sapphire.

In a preferred embodiment, when the polymerizable components in the fluid dispersion is composed essentially of prepolymers, reusable molds can be used. Examples of reusable molds made of quartz or glass are those disclosed in U.S. Patent No. 6,627,124. In this aspect, the fluid dispersion is poured into a mold consisting of two mold halves, the two mold halves not touching each other but having a thin gap of annular design arranged between them. The gap is connected to the mold cavity, so that excess prepolymer composition can flow into the gap. Instead of polypropylene molds that can be used only once, it is possible for reusable quartz, glass, sapphire molds to be used, since, following the production of a lens, these molds can be cleaned rapidly and effectively to remove unreacted materials and other residues, using water or a suitable solvent, and can be dried with air. Reusable molds can also be made of a cyclic olefin copolymer, such as for example, Topas® COC grade 8007-S10 (clear amorphous copolymer of ethylene and norbornene) from Ticona GmbH of Frankfurt, Germany and Summit, New Jersey, Zeonex® and Zeonor® from Zeon Chemicals LP, Louisville, KY. Because of the reusability of the mold halves, a relatively high outlay can be expended at the time of their production in order to obtain molds of extremely high precision and reproducibility. Since the mold halves do not touch each other in the region of the lens to be produced, i.e. the cavity or actual mold faces, damage as a result of contact is ruled out. This ensures a high service life of the molds, which, in particular, also ensures high reproducibility of the contact lenses to be produced and high fidelity to the lens design.

After the dispersion is dispensed into the mold, it is polymerized to produce a contact lens. Crosslinking and/or polymerizing may be initiated in the mold e.g. by means of actinic radiation, such as UV irradiation, ionizing radiation (e.g., gamma or X-ray irradiation). Where prepolymers of the invention are the polymerizable components in the fluid composition, the mold containing the fluid composition can be exposed to a spatial limitation of actinic radiation to crosslink the prepolymers.

Any known suitable methods can be used in the preparation of Ag-nanoparticles. For example, silver ions or silver salts can be reduced by means of a reducing agent (e.g., $NaBH_4$, ascorbic acid, citrate, or the like) or of heating or UV irradiation in a solution in the presence of a stabilizer to form Ag-nanoparticles. A person skilled in the art will know how to choose a suitable known method for preparing Ag-nanoparticles. The solution is then treated with chloride according to methods described above. Then, the prepared dispersion containing stabilized Ag-nanoparticles can be lyophilized (dry-freezed).

In accordance with this aspect of the invention, a polymerizable fluid composition can be a solution or a solvent-free liquid or melt at a temperature below 60°C.

In this aspect of the invention, the above described siloxane-containing macromers, siloxane-containing monomers, hydrophilic monomers, hydrophobic monomers, solvents, stabilizers for stabilizing Ag-nanoparticles, soluble silver salts, cross-linking agents, initiators, UV-absorbers, inhibitors, fillers, and visibility tinting agents can be used in preparation of a polymerizable fluid composition comprising a siloxane-containing macromer and a soluble silver salt. The formulations of soft contact lenses (such as lotrafilcon A, lotrafilcon B, etafilcon A, genfilcon A, lenefilcon A, polymacon, acquafilcon A, and balafilcon) can also be used.

Any one of the above described methods of the invention can be used to prepare an antimicrobial contact lens.

The molded contact lenses can further subject to one or more further processes, such as, for example, hydration, extraction, surface treatment, and the like, then placed in lens packages each containing a packaging solution, and sterilized the sealed lens packages with one lens therein. The packaging solution can comprise lubricants and/or viscosity adjusting agents, such as poly(vinyl alcohol) of a molecular weight of up to 1,500,000, polyvinylpyrrolidone of a molecular weight of up to 1,500,000, a copolymer (of a molecular weight of up to 1,500,000) of vinylpyrrolidone with another vinyl monomer, and the like.

Above described polymerizable fluid compositions can be used in the preparation of an antimicrobial contact lens according to any methods of the invention. The contact lenses preferably have a surface which is biocompatible with ocular tissue and ocular fluids during the desired extended period of contact.

In one preferred embodiment, the contact lenses include a core material, as defined above, surrounded, at least in part, by a surface which is more hydrophilic and lipophobic than the core material. A hydrophilic surface is desirable in order to enhance the compatibility of the lens with the ocular tissues and tear fluids. As surface hydrophilicity increases, undesirable attraction and adherence of lipids and proteinaceous matter typically decreases. There are factors other than surface hydrophilicity, such as immunological response, which may contribute to deposit accumulation on

the lens. Deposition of lipids and proteinaceous matter causes haze on the lens, thereby reducing visual clarity. Proteinaceous deposits may also cause other problems, such as irritation to the eye. After extended periods of continuous or intermittent wear, the lens must be removed from the eye for cleaning, i.e., deposit removal. Therefore, increased surface hydrophilicity, and concomitant reductions in deposits of biological matter, allows increased wear time.

There are a variety of methods disclosed in the art for rendering a surface of a material hydrophilic. For example, the lens may be coated with a layer of a hydrophilic polymeric material. Alternatively, hydrophilic groups may be grafted onto the surface of the lens, thereby producing a monolayer of hydrophilic material. These coating or grafting processes may be effected by a number of processes, including without limitation thereto, exposing the lens to plasma gas or immersing the lens in a monomeric solution under appropriate conditions.

Another set of methods of altering the surface properties of a lens involves treatment prior to polymerization to form the lens. For example, the mold may be treated with a plasma (i.e., an ionized gas), a static electrical charge, irradiation, or other energy source, thereby causing the prepolymerzation mixture immediately adjacent the mold surface to differ in composition from the core of the prepolymerization mixture.

A preferred class of surface treatment processes are plasma processes, in which an ionized gas is applied to the surface of an article. Plasma gases and processing conditions are described more fully in U.S. Patent Nos. 4,312,575 and 4,632,844. The plasma gas is preferably a mixture of lower alkanes and nitrogen, oxygen or an inert gas.

In a preferred embodiment, a contact lens is subjected to a plasma treatment in the presence of a mixture of (a) a $C_{1-6}$ alkane and (b) a gas selected from the group consisting of nitrogen, argon, oxygen, and mixtures thereof. In a more preferred embodiment, the lens is plasma treated in the presence of a mixture of methane and air.

In another preferred embodiment, a contact lens has an LbL coating thereon. Formation of an LbL coating on a contact lens may be accomplished in a number of ways, for example, as described in US Patent Ser. No. 6,451,871 and pending U.S. patent applications (Appl. Nos. 09/774942, 09/775104, 60/409,950). One coating process embodiment involves solely dip-coating and dip-rinsing steps. Another coating process embodiment involves solely spray-coating and spray-rinsing steps. However, a number of alternatives involve various combinations of spray- and dip-coating and rinsing steps may be designed by a person having ordinary skill in the art.

The previous disclosure will enable one having ordinary skill in the art to practice the invention. In order to better enable the reader to understand specific embodiments and the advantages thereof, reference to the following examples is suggested.

**Example 1**

Unless otherwise stated, all chemicals are used as received. Oxygen and ion permeability measurements are carried out with lenses after extraction and plasma coating. Non-plasma coated lenses are used for tensile testing and water content measurements.

**Oxygen permeability measurements.** The oxygen permeability of a lens and oxygen transmissibility of a lens material is determined according to a technique similar to the one described in U.S. Patent No. 5,760,100 and in an article by Winterton et al., (The Cornea: Transactions of the World Congress on the Cornea 111, H.D. Cavanagh Ed., Raven Press: New York 1988, pp273-280), both of which are herein incorporated by reference in their entireties. Oxygen fluxes (J) are measured at 34°C in a wet cell (i.e., gas streams are maintained at about 100% relative humidity) using a Dk1000 instrument (available from Applied Design and Development Co., Norcross, GA), or similar analytical instrument. An air stream, having a known percentage of oxygen (e.g., 21%), is passed across one side of the lens at a rate of about 10 to 20 $cm^3$ /min., while a nitrogen stream is passed on the opposite side of the lens at a rate of about 10 to 20 $cm^3$/min. A sample is equilibrated in a test media (i.e., saline or distilled water) at the prescribed test temperature for at least 30 minutes prior to measurement but not more than 45 minutes. Any test media used as the overlayer is equilibrated at the prescribed test temperature for at least 30 minutes prior to measurement but not more than 45 minutes. The stir motor's speed is set to 1200±50 rpm, corresponding to an indicated setting of 400±15 on the stepper motor controller. The barometric pressure surrounding the system, $P_{measured}$, is measured. The thickness (t) of the lens in the area being exposed for testing is determined by measuring about 10 locations with a Mitotoya micrometer VL-50, or similar instrument, and averaging the measurements. The oxygen concentration in the nitrogen stream (i.e., oxygen which diffuses through the lens) is measured using the DK1000 instrument. The apparent oxygen permeability of the lens material, $Dk_{app}$, is determined from the following formula:

$$Dk_{app} = Jt/(P_{oxygen})$$

where

J=oxygen flux [microliters $O_2$/$cm^2$ -minute]
$P_{oxygen}$ =($P_{measured}$ -$P_{water}$ vapor)=(%$O_2$ in air stream) [mm Hg]=partial pressure of oxygen in the air stream
$P_{measured}$ =barometric pressure (mm Hg)
$P_{water}$ vapor =0 mm Hg at 34 °C (in a dry cell) (mm Hg)
$P_{water}$ vapor =40 mm Hg at 34°C (in a wet cell) (mm Hg)
t=average thickness of the lens over the exposed test area (mm)

where $Dk_{app}$ is expressed in units of barrers.

The oxygen transmissibility (Dk/t) of the material may be calculated by dividing the oxygen permeability ($Dk_{app}$) by the average thickness (t) of the lens.

**Ion Permeability Measurements.** The ion permeability of a lens is measured according to procedures described in U.S. Patent No. 5,760,100. The values of ion permeability reported in the following examples are relative ionoflux diffusion coefficients ($D/D_{ref}$) in reference to a lens material, Alsacon, as reference material Alsacon has an ionoflux diffusion coefficient of $0.314X10^{-3}$ $mm^2$/minute.

**Antimicrobial Activity Assay**

Antimicrobial activity of some contact lenses with or without silver nanoparticles in the lenses of the invention is also assayed against *Staphylococcus aureus* ATCC #6538. Bacterial cells of *S. aureus* #6538 are stored in a lyophilized state. Bacteria are grown on a Tryptic Soy agar slant for 18 hours at 37 °C. The cells are harvested by centrifugation and washed twice with sterile, Delbeco's phosphate buffered saline. Bacterial cells are suspended in 1/20 th strength Tryptic Soy Broth (TSB) and adjusted to Optical Density of $10^8$ cfu. The cell suspension is serially diluted to $10^3$ cfu/ml in 1/20th strength TSB.

Lenses having a silver in them are tested against the control lenses (i.e., without a silver). 200μl of from about $5x10^3$ to $1x10^4$ cfu/ml of *S. aureus* #6538 is placed on the surface of each lens. Incubate at 25°C for 24 hours. Aspirate 50 μl out of the lens, serially dilute and plate out on agar plates to determine the microbial load of each lens. At 24 hours, colony counts are taken.

**Example 2**

**Synthesis of Macromer**

51.5g (50 mmol) of the perfluoropolyether Fomblin® ZDOL (from Ausimont S.p.A, Milan) having a mean molecular weight of 1030 g/mol and containing 1.96meq/g of hydroxyl groups according to end-group titration is introduced into a three-neck flask together with 50mg of dibutyltin dilaurate. The flask contents are evacuated to about 20 mbar with stirring and subsequently decompressed with argon. This operation is repeated twice. 22.2g (0.1 mol) of freshly distilled isophorone diisocyanate kept under argon are subsequently added in a counterstream of argon. The temperature in the flask is kept below 30°C by cooling with a waterbath. After stirring overnight at room temperature, the reaction is complete. Isocyanate titration gives an NCO content of 1.40 meq/g (theory: 1.35 meq/g).

202g of the α,ω-hydroxypropyl-terminated polydimethylsiloxane KF-6001 from Shin-Etsu having a mean molecular weight of 2000g/mol (1.00meq/g of hydroxyl groups according to titration) are introduced into a flask. The flask contents are evacuated to approx. 0.1 mbar and decompressed with argon. This operation is repeated twice. The degassed siloxane is dissolved in 202ml of freshly distilled toluene kept under argon, and 100mg of dibutyltin dilaurate (DBTDL) are added. After complete homogenization of the solution, all the perfluoropolyether reacted with isophorone diisocyanate (IPDI) is added under argon. After stirring overnight at room temperature, the reaction is complete. The solvent is stripped off under a high vacuum at room temperature. Microtitration shows 0.36meq/g of hydroxyl groups (theory 0.37meq/g). 13.78g (88.9mmol) of 2-isocyanatoethyl methacrylate (IEM) are added under argon to 247g of the α,σ-hydroxypropyl-terminated polysiloxane-perfluoropolyether-polysiloxane three-block copolymer (a three-block copolymer on stoichiometric average, but other block lengths are also present). The mixture is stirred at room temperature for three days. Microtitration then no longer shows any isocyanate groups (detection limit 0.01meq/g). 0.34meq/g of methacryl groups are found (theory 0.34meq/g).

The macromer prepared in this way is completely colourless and clear. It can be stored in air at room temperature for several months in the absence of light without any change in molecular weight.

**Example 3**

A lens-forming formulation (polymerizable dispersion) containing Ag-nanoparticles and copper phthalocyanin (CuP) particles is prepared by mixing appropriate amount of following components: about 37.5% by weight of macromer

prepared in Example 2, about 60 ppm CuP (copper phthalocyanin), about 15% by weight of TRIS, about 22.5% by weight of DMA, about 24.8% by weight of ethanol and about 0.2% by weight of **Darocure® 1173.** CuP particles are added into the formulation by adding appropriate amount of CuP pigment stock dispersion in Tris, which is prepared by diluting more concentrated CuP-Tris suspension to lower concentration of CuP using TRIS.

As described in commonly owned co-pending U.S. patent application publication No. 2005/0013842A1, silver particles are formed in the formulation by using appropriate silver source or silver salt and stabilizer. For example, a silver stock solution (SSS) is prepared by adding appropriate amount of silver salt (e.g. silver nitrate), stabilizer (e.g., acrylic acid) into DMA. Then appropriate amount of SSS is then added to the formulation with blue pigment, preferably with the final $AgNO_3$ concentration of at least 0.003%, or even more preferably at least 0.01%. The formulation is mixed thoroughly by stirring or rolling and stored at room temperature overnight to allow the formation of silver nanoparticles, as indicated by the color change from blue to greenish blue. Note that not all silver salt can be used, for example, silver acetate has a low solubility in DMA solution containing stabilizer and therefore is not a preferred silver source.

**Example 4**

**Chloride Treatment**

**A.** 0.068g sodium chloride (NaCl) solid is added to 100g of the formulation prepared in Example 3. White sodium chloride solid can be seen in the bottom of the vial. The color appearance of the formulation is monitored overtime. No color change is observed up to at least 1 hr after adding solid NaCl. After overnight (~18 hr or more), the color of the formulation changes from blue-greenish to blue. The formulation is then filtered to collect the NaCl solid. The collected NaCl solid is then dissolved in 10 ml of water and analyzed by atomic adsorption (AA) for silver concentration. About 2.2 ppm of silver is detected. Using the formulation prepared in Example 3 as reference, the color change is also monitored by by CMC tolerancing using X-Rite SP64 Spectrophotometer. CMC tolerancing is developed by the Color Measurement Committee of the Society of Dyers and Colorists in Great Britain and became public domain in 1988. The measured value is the CMC difference between the test sample and arbitrarily chosen reference sample. The small the CMC difference, the closer of the color of the test sample as compared to the reference sample. About ~1hr after adding NaCl, the x-rite measurement (CMC tolerancing) for the formulation is 8.5. After overnight, it increases to ~14. After 2 days, it is about ~13.8. After another 11 days, it is 13.4. when measured again in 31 days, it is 13.6. The change in x-rite measurement is in agreement with the color change after adding NaCl.

**B.** 0.34g sodium chloride (NaCl) solid is added to 100g of the formulation prepared in Example 3. White sodium chloride solid can be seen in the bottom of the vial. The color appearance of the formulation is monitored overtime. No color change is observed up to at least 1 hr after adding NaCl. After overnight (~18 hr or more), the color of the formulation changes from blue-greenish to blue. The formulation is then filtered to collect the NaCl solid. The solid is then dissolved in 10 ml of water and analyzed by atomic adsorption (AA) for silver concentration. About 3.9ppm of silver is detected. The color change is also monitored by x-rite measurement by using the formulation prepared in Example 3 as reference. About ~1hr after adding NaCl, the x-rite measurement for the formulation is 8.3. After overnight, it increases to ~14. After 2 days, it is about ~13.1. After another 11 days, it is 13.6. when measured again in 31 days, it is 13.1. The change in x-rite measurement is in agreement with the color change after adding NaCl.

**C.** To prepare an ethanol solution containing hydrochloride (HCl), 0.76g of concentrated hydrochloride acid (36.4% of HCl in water) is added to 9.24g of ethanol. 0.5g of the ethanol containing HCl is added to 100g of the formulation prepared in Example 3. The color appearance of the formulation is monitored overtime. Color change from blue-greenish to blue is observed at ~ 1 hr after adding HCl ethanol solution. The color change is also monitored by x-rite measurement by using the formulation prepared in Example 3 as reference. About ~1hr after adding HCl, the x-rite measurement for the formulation is 15.2. After overnight, it is measured as ~16. After 2 days, it is about ~15.4. After another 11 days, it is 15.7. When measured again in 31 days, it is ~15. The change in x-rite measurement is in agreement with the color change after adding NaCl.

**Example 5**

**Lens preparation**

**A.** An amount of the formulation in Example 4A is introduced into each polypropylene molds and cured for 6 minutes under UV light to form contact lenses. The lenses are then extracted in isopropyl alcohol (IPA), then packaged and

autoclaved in phosphate buffered saline. The silver concentration in the lens is determined to be around 135ppm, as measured by instrumental neutron activation analysis (INAA).

**B.** An amount of the formulation in Example 4B is introduced into each polypropylene molds and cured for 6 minutes under UV light to form contact lenses. The lenses are then extracted in isopropyl alcohol (IPA), then packaged and autoclaved in phosphate buffered saline. The silver concentration in the lens is determined to be around 133ppm, as measured by instrumental neutron activation analysis (INAA).

**C.** An amount of the formulation in Example 4C is introduced into each polypropylene molds and cured for 6 minutes under UV light to form contact lenses. The lenses are then extracted in isopropyl alcohol (IPA), then packaged and autoclaved in phosphate buffered saline. The silver concentration in the lens is determined to be around 121ppm, as measured by instrumental neutron activation analysis (INAA).

**D.** The formulation in Example 4C is degassed to remove oxygen from the formulation. An amount of the degassed formulation is introduced into each polypropylene molds in a nitrogen glove box and cured for 6 minutes under UV light to form contact lenses. The lenses are then extracted in isopropyl alcohol (IPA), then packaged and autoclaved in phosphate buffered saline. The average silver concentration in the lenses is determined to be around 80ppm, as measured by instrumental neutron activation analysis (INAA).

Lenses in Example 5C are made from non-degassed formulation and have ion permeability (IP) less than 1.0. Lenses in Example 5D are made from degassed formulation and have ion permeability (IP) greater than 1.0. Lower silver concentration in lenses from Example 10 indicate more silver loss during the IPA extraction and water hydration steps for lenses with higher IP.

**Example 6**

**Antimicrobial Activity Assay**

Antimicrobial activity of contact lenses with silver nanoparticles prepared in Examples 5A, 5B, and 5C is assayed against *S. aureus* #6538 according to the procedure described in Example 1. All lenses show antimicrobial activity, characterized by at least 98.0%, 98.1% and 98.9% inhibition of viable cells as compared to the control lenses, for the three lots of lenses from Examples 5A, 5B, and 5C, respectively.

**Example 7**

**Silver concentration in formulations and the impact of filtration**

The formulation is prepared as described in Example 4C. Then the formulation is filtered using a 5 micro membrane filter. As measured by Instrumental Neutron Activation Analysis (INAA), the silver concentration for un-filtered formulation is about 154 ppm. After filtration, the silver concentration in formulation is about 104 ppm. Regarding to maintain silver concentration in formulation, filtration is not preferred. After filtration, the formulation is degassed to remove oxygen from the formulation. The silver concentration in degassed formulation is measured as about 108 ppm.

**Example 8**

**Particle size and silver concentration of different formulations.**

A series of formulations similar to the one described in Example 4C are prepared with following two variations:

(1) by changing the silver to chloride (Ag/Cl) ratio
(2) by using ethanol containing hydrochloride but not water (instead of adding concentrated aqueous hydrochloride to ethanol as in Example 4C).

The particles of these formulations are studied by placing a drop of formulation on a cover glass slide and using a high magnificent light microscope. Certain degrees of particle aggregation are observed for all formulations. As listed in Table 1, when visualized under 1000x, particles aggregations of 2 to 7 individual particles are observed. The averaged apparent particle size from ~30 data points is also listed. The preferred Ag/Cl ratio is 1:1 to 1:6.

Table 1

| HCl resource | [Ag]/[Cl] | Particle aggregation observations | Estimated Average particle size in micron# |
|---|---|---|---|
| Ethanol + HCl (36.4%) | 1:0.5 | 2-12 particles | Not measured |
| Ethanol + HCl (36.4%) | 1:1 | 2-6 particles | 2.0 |
| Ethanol + HCl (36.4%) | 1:2 | 2-5 particles | 2.2 |
| Ethanol + HCl (36.4%) | 1:4 | 2-5 particles | 2.1 |
| Ethanol + HCl (36.4%) | 1:6 | 2-7 particles | 1.8 |
| Ethanol + HCl (36.4%) | 1:2 | 2-7 particles | 2.0 |
| Ethanol with HC1 (1.25M)* | 1:4 | 2-6 particles | 2.1 |
| Ethanol with HC1 (1.25M)* | | 2-6 particles | 2.3 |
| * from Fluka<br># without turning on the ultra-sonication function of the particle size analyzer | | | |

**Example 9**

Instead of using hydrochloride in ethanol, other compounds, such as, hydroiodic acid (HI), phosphoric acid ($H_3PO_4$), oxalic acid (HOOCCOOH), was tested. As mentioned previously, the color of the formulation will change from bluish green to blue after the addition of HCl. However, after the addition of phosphoric acid and oxalic acid, the formulation appears to have minimum color change. After the addition of HI, the color of the formulation changed to blue. However, under the normal curing time (6 minutes) as used for other formulation, the HI-containing formulation did not cured into lens, although longer cure time (e.g. 60 min) is able to cure the formulation into lens.

**Example 10**

Instead of using hydrochloride in ethanol as described in Example 4C, hydrochloride is first dissolved in a small amount of DMA. Two formulations are then prepared according to the procedure described in Example 4C, except by adding hydrochloride/DMA solution either before or after the SSS. As measured by particle size analyzer Horiba LA-920, without turning on the ultra-sonication function of the particle size analyzer, the mean particle size is estimated ~3 microns when HCl/DMA is added before SSS (formulation 10a), as compared to about 2 microns when HC1/DMA is added after SSS (formulation 10b).

The oxygen in the formulation is removed by vacuum degas. An amount of the formulation is introduced into each polypropylene molds and cured for 6 minutes under UV light to form contact lenses. The lenses are then extracted in isopropyl alcohol (IPA), then packaged and autoclaved in phosphate buffered saline. The particle sizes in lenses are estimated by using high magnification light microscope (e.g. X500 or X1000). For lenses made from formulation 10a, the estimated average particles size is about 4 microns. And for lenses made from formulation 10b, it is about 2.5 microns. As measured by instrumental neutron activation analysis (INAA), the silver concentration in the lens from formulation 10a is determined to be around 173 ppm , as compared to 44 ppm in the lens from formulation 10b. Clearly, the procedure or order of adding HCl/DMA will impact the final lens properties.

**Example 11**

Instead of using hydrochloride as described in Example 4C, a reducing agent is used in this example. The reducing agent used in the experiments is Borane-dimethylamine complex (BDC). Similar to Example 10, two formulations are then prepared according to the procedure described in Example 4C, except by adding BDC either before (formulation 11a) or after the SSS (formulation 11b). As measured by particle size analyzer Horiba LA-920, without turning on the ultra-sonication function of the particle size analyzer, the mean particle size is estimated ~1.8 microns when BDC is added before SSS (formulation 11a), as compared to about 2 microns when BDC is added after SSS (formulation 11b).

The oxygen in the formulation is removed by vacuum degas. An amount of the formulation is introduced into each polypropylene molds and cured for 6 minutes under UV light to form contact lenses. The lenses are then extracted in isopropyl alcohol (IPA), then packaged and autoclaved in phosphate buffered saline. The particle sizes in lenses are

estimated by using high magnification light microscope (e.g. X500 or X1000). For lenses made from formulation 11a, the estimated average particles size is about 1.6 microns. And for lenses made from formulation 11b, it is about 2.2 microns. As measured by instrumental neutron activation analysis (INAA), the silver concentration in the lens from formulation 11a is determined to be around 134 ppm , as compared to 132 ppm in the lens from formulation 11b. The order of adding BDC seems have little impact on the final lens properties.

In addition to BDC, another example of reducing agent can be used is ascorbic acid (or vitamin C). However, it is important to point out that not all reducing agent can be used in the formulation. For example, sodium borohydride ($NaBH_4$), a widely know reducing agent used extensively in published literatures to reduce silver ions to silver nanoparticles, cannot be used in this case, because it causes violent polymerization of DMA.

**Example 12**

Both BDC and HCl can be added to the same formulation. As an example, a formulation is prepared by added DBC before adding SSS. Then HCl in DMA is added after adding SSS. Using the same procedure and testing methods as in Examples 10 and 11, the mean particle size in the formulation is estimated to be about 5.7 microns. The average particle size in the lens is estimated to be about 4.3 microns (without turning on the ultra-sonication function of the particle size analyzer). The silver concentration in the lens is estimated to be 85 ppm. Although in this experiments, the particles size increases as compared to the data in Examples 10 and 11, it is possible to manipulate the particle size and silver concentration by controlling the procedure and order of adding BDC and HCl, and/or by controlling the relative ratio of silver to BDC or to HCl.

**Example 13**

**in-vitro antimicrobial activity of lenses from Example 10, 11 and 12.**

Antimicrobial activity of contact lenses with silver nanoparticles is assayed against *S. aureus* #6538 according to the procedure described in Example 6. All lenses show antimicrobial activity, characterized by at least 99.8% to 99.9% inhibition of viable cells as compared to the control lenses.

**Example 14**

Silver release from the lenses into phosphate buffered saline (PBS). The silver release from the lenses is studied by measuring the silver concentration in PBS after incubating the lenses in PBS for certain period of time.

The detailed procedure is described as following: Remove lenses from containers and save packaging saline for Ag analysis. Rinse lenses with sterile blank saline solution and blot dry with Kim Wipes. Place one lens each in plastic vials and add 1.5 ml sterile saline solution. Swirl vial to assure that the lenses unfold concave side up in the vial. Place the vials with lenses in a 35 ± 2 °C oven for twenty-four hours. At the end of 24 hours, remove the saline from the vials and save for Ag analysis. Add 1.5 ml fresh saline to the lenses and return the vials to the oven. Repeat the saline exchange step for the desired number of days. The silver release into PBS or silver concentration in PBS was then analyzed by graphite furnace atomic absorption (GFAA).

Along with a control sliver lenses made from formulation without adding BDC or HCl, lenses made from Examples 10, 11 and 12 are studied for silver release. As shown in Table 2 for data up to 4 days of release study, adding HCl, especially before adding SSS, significantly increased the release of silver from the lenses. The increased release of silver may lead to better anti-bacterial activity, although it may also impose challenge in controlling silver loss during wet process steps when making lenses.

Table 2. Silver concentration in PBS (ppb)

| days | Control | Adding HCl before adding SSS | Adding HCl after adding SSS | Adding BDC before adding SSS | Adding BDC after adding SSS | Adding both BDC and HCl |
|---|---|---|---|---|---|---|
| 1 | 13.0 | 101.0 | 49.0 | 9.5 | 16.0 | 18.5 |
| 2 | 6.4 | 77.4 | 27.5 | 5.2 | 9.8 | 12.1 |
| 3 | 6.4 | 77.4 | 24.0 | 6.4 | 11.0 | 13 |
| 4 | 6.2 | 55.0 | 16.8 | 6 | 10.4 | 11.3 |

## Claims

1. A method for making an antimicrobial contact lens, which comprises the steps of:

    introducing an amount of a polymerizable dispersion in a mold for making a contact lens; and
    polymerizing the polymerizable dispersion in the mold to form the antimicrobial contact lens;
    **characterized in that** the polymerizable dispersion is prepared as follows:

        obtaining a polymerizable dispersion comprising *in-situ* formed Ag-nanoparticles and a silicone-containing monomer or macromer or prepolymer;
        treating the Ag-nanoparticles-containing polymerizable dispersion with chloride.

2. The method of claim 1, wherein *in-situ* formation of Ag-nanoparticles is performed according to either process A or process B or combination thereof,
wherein the process A comprises the steps of: (1) adding a desired amount of a soluble silver salt into a polymerizable fluid composition comprising a monomer capable of reducing silver cations and a silicone-containing monomer or macromer or prepolymer so as to form a mixture; and (2) mixing thoroughly the mixture for a period of time long enough to reduce at least 20% of the added amount of the silver salt into silver nanoparticles so as to form a polymerizable dispersion, and wherein the process B comprises the steps of: (1) adding into a polymerizable fluid composition comprising a silicone-containing monomer or macromer or prepolymer at least one biocompatible reducing agent to form a mixture, wherein the amount of the biocompatible reducing agent added in the mixture is sufficient to reduce at least 20% of the silver salt into Ag-nanoparticles; and (2) mixing thoroughly the mixture for a period of time sufficient to reduce at least 20% of the silver salt into Ag-nanoparticles so as to form a polymerizable dispersion.

3. The method of claim 1, wherein the step of treating the Ag-nanoparticles-containing polymerizable dispersion with chloride is performed according to either procedure A or procedure B or combination thereof,
wherein the procedure A comprises: (1) adding chloride salt, such as NaCl in solid form, directly into the dispersion; (2) mixing thoroughly resultant mixture for a period of time long enough to substantially reduce yellowish color of Ag-nanoparticles in the dispersion; and (3) removing remaining solid chloride salt,
wherein the procedure B comprises: (1) adding a NaCl or hydrochloride solution into the dispersion and (2) mixing thoroughly resultant mixture for a period of time long enough to substantially reduce yellowish color of Ag-nanoparticles in the dispersion.

4. The method of claim 1, 2 or 3, wherein the silicone-containing prepolymer is at least one member selected from the group consisting of a prepolymer with at least one ethylenically unsaturated group, a prepolymer with two or more thiol groups, a prepolymer with at least one ene-group; wherein the silicone-containing macromer is at least one member selected from the group consisting of a macromer with at least one ethylenically unsaturated group, a macromer with two or more thiol groups, a macromer with at least one ene-group; wherein the silicone-containing monomer is at least one member selected from the group consisting of a monomer with one ethylenically unsaturated group, a monomer with two thiol groups, a monomer with one ene-group; wherein the ene-group is defined by any one of formula (I) - (III)

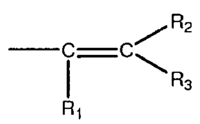

(I)

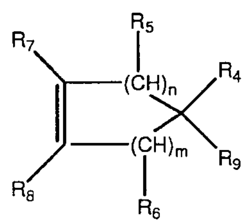

(II)

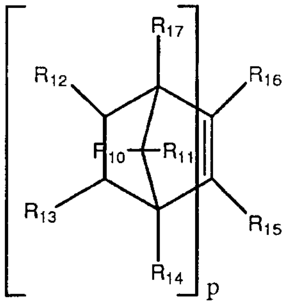

(III)

in which $R_1$ is hydrogen, or $C_1$-$C_{10}$ alkyl; $R_2$ and $R_3$ independent of each other are hydrogen, $C_1$-$C_{10}$ alkene divalent radical, $C_1$-$C_{10}$ alkyl, or $-(R_{18})_a-(X_1)_b-R_{19}$ in which $R_{18}$ is $C_1$-$C_{10}$ alkene divalent radical, $X_1$ is an ether linkage (-O-), a urethane linkage (-N), a urea linkage, an ester linkage, an amid linkage, or carbonyl, $R_{19}$ is hydrogen, a single bond, amino group, carboxylic group, hydroxyl group, carbonyl group, $C_1$-$C_{12}$ aminoalkyl group, $C_1$-$C_{18}$ alkylaminoalkyl group, $C_1$-$C_{18}$ carboxyalkyl group, $C_1$-$C_{18}$ hydroxyalkyl group, $C_1$-$C_{18}$ alkylalkoxy group, $C_1$-$C_{12}$ aminoalkoxy group, $C_1$-$C_{18}$ alkylaminoalkoxy group, $C_1$-$C_{18}$ carboxyalkoxy group, or $C_1$-$C_{18}$ hydroxyalkoxy group, a and b independent of each other is zero or 1, provided that only one of $R_2$ and $R_3$ is a divalent radical; $R_4$ - $R_9$, independent of each other, are hydrogen, $C_1$-$C_{10}$ alkene divalent radical, $C_1$-$C_{10}$ alkyl, or $-(R_{18})_a-(X_1)_b-R_{19}$, optionally $R_4$ and $R_9$ are linked through an alkene divalent radical to form a cyclic ring, provided that at least one of $R_4$ - $R_9$ are divalent radicals; n and m independent of each other are integer number from 0 to 9, provided that the sum of n and m is an integer number from 2 to 9; $R_{10}$ - $R_{17}$, independent of each other, are hydrogen, $C_1$-$C_{10}$ alkene divalent radical, $C_1$-$C_{10}$ alkyl, or $-(R_{18})_a-(X_1)_b-R_{19}$, p is an integer number from 1 to 3, provided that only one or two of $R_{10}$ - $R_{17}$ are divalent radicals.

## Patentansprüche

**1.** Verfahren zur Herstellung einer antimikrobiellen Kontaktlinse, umfassend die Schritte:

Einbringen einer Menge einer polymerisierbaren Dispersion in eine Form zur Herstellung einer Kontaktlinse; und
Polymerisieren der polymerisierbaren Dispersion in der Form zur Bildung der antimikrobiellen Kontaktlinse;
**dadurch gekennzeichnet, dass** die polymerisierbare Dispersion wie folgt hergestellt wird:
Erhalten einer polymerisierbaren Dispersion enthaltend *in-situ*-gebildete Ag-Nanopartikel und ein Silikon-enthaltendes Monomer oder Makromer oder Präpolymer;
Behandeln der Ag-Nanopartikel-enthaltenden polymerisierbaren Dispersion mit Chlorid.

**2.** Verfahren gemäss Anspruch 1, wobei die *in-situ*-Bildung der Ag-Nanopartikel entweder gemäss Verfahren A oder Verfahren B oder einer Kombination davon durchgeführt wird,
wobei das Verfahren A die Schritte umfasst: (1) Zugeben einer gewünschten Menge eines löslichen Silbersalzes in eine polymerisierbare Fluid-Zusammensetzung enthaltend ein Monomer das in der Lage ist Silberkationen zu reduzieren und ein Silikon-enthaltendes Monomer oder Makromer oder Präpolymer zur Bildung einer Mischung; und (2) sorgfältiges Durchmischen der Mischung für eine Zeitdauer die lang genug ist um mindestens 20% der zugegebenen Menge des Silbersalzes zu Silber-Nanopartikeln zu reduzieren um so eine polymerisierbare Dispersion zu bilden, und
wobei das Verfahren B die Schritte umfasst: (1) Zugeben mindestens eines bioverträglichen Reduktionsmittels in eine polymerisierbare Fluid-Zusammensetzung enthaltend ein Silikon-enthaltendes Monomer oder Makromer oder Präpolymer zur Bildung einer Mischung, wobei die Menge des zur Mischung zugegebenen bioverträglichen Reduktionsmittels ausreichend ist um mindestens 20% des Silbersalzes zu Ag-Nanopartikeln zu reduzieren; und (2) sorgfältiges Durchmischen der Mischung für eine Zeitdauer die lang genug ist um mindestens 20% der zugegebenen Menge des Silbersalzes zu Silber-Nanopartikeln zu reduzieren um so eine polymerisierbare Dispersion zu bilden.

**3.** Verfahren gemäss Anspruch 1, wobei der Schritt des Behandelns der Ag-Nanopartikel-enthaltenden polymerisierbaren Dispersion mit Chlorid entweder gemäss Verfahren A oder Verfahren B oder einer Kombination davon durchgeführt wird,
wobei das Verfahren A umfasst: (1) Zugeben von Chlorid-Salz, wie etwa NaCl in fester Form, direkt in die Dispersion; (2) gründliches Durchmischen der erhaltenen Mischung für eine Zeitdauer die lang genug ist um gelbliche Farbe der Ag-Nanopartikel in der Dispersion wesentlich zu reduzieren; und (3) Entfernen von zurückgebliebenem festem

Chlorid-Salz,

wobei das Verfahren B umfasst: (1) Zugeben einer NaCl- oder Hydrochlorid-Lösung in die Dispersion und (2) sorgfältiges Durchmischen der erhaltenen Mischung für eine Zeitdauer die lang genug ist um gelbliche Farbe der Ag-Nanopartikel in der Dispersion wesentlichen zu reduzieren.

**4.** Verfahren gemäss Anspruch 1, 2 oder 3, wobei das Silikon-enthaltende Präpolymer mindestens ein Mitglied ausgewählt aus der Gruppe bestehend aus einem Präpolymer mit mindestens einer ethylenisch-ungesättigten Gruppe, einem Präpolymer mit zwei oder mehr Thiol-Gruppen, einem Präpolymer mit mindestens einer En-Gruppe ist; wobei das Silikon-enthaltende Makromer mindestens ein Mitglied ausgewählt aus der Gruppe bestehend aus einem Makromer mit mindestens einer ethylenisch-ungesättigten Gruppe, einem Makromer mit zwei oder mehr Thiol-Gruppen, einem Makromer mit mindestens einer En-Gruppe ist; wobei das Silikon-enthaltende Monomer mindestens ein Mitglied ausgewählt aus der Gruppe bestehend aus einem Monomer mit einer ethylenisch-ungesättigten Gruppe, einem Monomer mit zwei Thiol-Gruppen, einem Monomer mit einer En-Gruppe ist; wobei die En-Gruppe durch eine der Formeln (I) - (III) definiert ist,

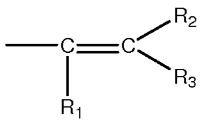
(I)

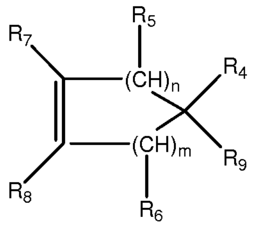
(II)

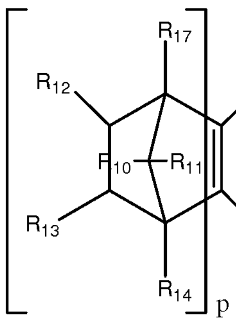
(III)

in welcher $R_1$ Wasserstoff oder $C_1$-$C_{10}$ Alkyl ist; $R_2$ und $R_3$ unabhängig voneinander Wasserstoff, zweiwertiger $C_1$-$C_{10}$ Alken-Rest, $C_1$-$C_{10}$ Alkyl oder-$(R_{18})_a$-$(X_1)_b$-$R_{19}$ sind, wobei $R_{18}$ ein zweiwertiger $C_1$-$C_{10}$ Alken-Rest ist, $X_1$ eine Ether-Bindung (-O-), eine Urethan-Bindung (-N), eine Harnstoff-Bindung, eine Ester-Bindung, eine AmidBindung, oder Carbonyl ist, $R_{19}$ Wasserstoff, eine Einfachbindung, Amino-Gruppe, Carboxy-Gruppe, Hydroxy-Gruppe, Carbonyl-Gruppe, $C_1$-$C_{12}$ Aminoalkyl-Gruppe, $C_1$-$C_{18}$ Alkyl-Aminoalkyl-Gruppe, $C_1$-$C_{18}$ Carboxyalkyl-Gruppe, $C_1$-$C_{18}$ HydroxyalkylGruppe, $C_1$-$C_{18}$ Alkylalkoxy-Gruppe, $C_1$-$C_{12}$ Aminoalkoxy-Gruppe, $C_1$-$C_{18}$ AlkylAminoalkoxy-Gruppe, $C_1$-$C_{18}$ Carboxyalkoxy-Gruppe, oder eine $C_1$-$C_{18}$ Hydroxyalkoxy-Gruppe ist, a und b unabhängig voneinander Null oder 1 sind, vorbehaltlich dass nur einer der $R_2$ und $R_3$ ein zweiwertiger Rest ist; $R_4$ - $R_9$ unabhängig voneinander Wasserstoff, ein zweiwertiger $C_1$-$C_{10}$ Alken-Rest, $C_1$-$C_{10}$ Alkyl oder-$(R_{18})_a$-$(X_1)_b$-$R_{19}$ sind, $R_4$ und $R_9$ wahlweise durch einen zweiwertigen Alken-Rest zur Bildung eines Rings verbunden sind, vorbehaltlich dass mindestens einer der $R_4$ - $R_9$ ein zweiwertiger Rest ist; n und m unabhängig voneinander ganze Zahlen von 0 bis 9 sind, vorbehaltlich dass die Summe aus n und m eine ganze Zahl von 2 bis 9 ist; $R_{10}$ - $R_{17}$ unabhängig voneinander Wasserstoff, ein zweiwertiger $C_1$-$C_{10}$ Alken-Rest, $C_1$-$C_{10}$ Alkyl oder -$(R_{18})_a$-$(X_1)_b$-$R_{19}$ sind, p eine ganze Zahl von 1 bis 3 ist, vorbehaltlich dass nur eines oder zwei der $R_{10}$ - $R_{17}$ zweiwertige Reste sind.

## Revendications

**1.** Procédé de fabrication d'une lentille de contact antimicrobienne, comprenant les étapes qui consistent à :

introduire une quantité d'une dispersion polymérisable dans un moule destiné à la fabrication d'une lentille de contact et à
polymériser la dispersion polymérisable dans le moule afin de former la lentille de contact antimicrobienne

**caractérisé en ce que** l'on prépare la dispersion polymérisable de la façon suivante :

obtention d'une dispersion polymérisable comprenant des nanoparticules d'Ag formées *in situ* et un monomère ou macro mère ou prépolymère contenant de la silicone ;
traitement de la dispersion polymérisable contenant des nanoparticules d'Ag avec du chlorure.

**2.** Procédé selon la revendication 1, dans lequel on réalise la formation de nanoparticules d'Ag *in situ* conformément à soit le processus A, soit le processus B, soit une combinaison de ceux-ci, le processus A comprenant les étapes qui consistent à : (1) ajouter une quantité souhaitée d'un sel d'argent soluble dans une composition fluide polymérisable comprenant un monomère présentant la faculté de réduire les cations argent et un monomère ou macromère ou prépolymère contenant de la silicone de façon à former un mélange ; et à (2) mélanger le mélange intimement pendant une période de temps suffisamment longue pour transformer, par réduction, au moins 20 % de la quantité du sel d'argent ajoutée en nanoparticules d'argent de façon à former une dispersion polymérisable, et le processus B comprenant les étapes qui consistent à : (1) ajouter, dans une composition fluide polymérisable comprenant un monomère ou macromère ou prépolymère contenant de la silicone, au moins un réducteur biocompatible afin de former un mélange, la quantité de réducteur biocompatible ajouté dans le mélange étant suffisante pour transformer, par réduction, au moins 20 % du sel d'argent en nanoparticules d'Ag ; et à (2) mélanger le mélange intimement pendant une période de temps suffisante pour transformer, par réduction, au moins 20 % du sel d'argent en nanoparticules d'Ag de façon à former une dispersion polymérisable.

**3.** Procédé selon la revendication 1, dans lequel l'étape qui consiste à traiter la dispersion polymérisable contenant des nanoparticules d'Ag avec du chlorure est réalisée au choix, soit selon la procédure A, soit selon la procédure B, soit selon une combinaison de celles-ci,
la procédure A comprenant les étapes qui consistent à : (1) ajouter un sel chlorure, tel que du NaCl sous forme solide, directement à la dispersion ; à (2) mélanger intimement le mélange qui en résulte pendant une période de temps suffisamment longue pour atténuer sensiblement la couleur jaunâtre des nanoparticules d'Ag dans la dispersion ; et à (3) séparer le sel chlorure solide restant,
la procédure B comprenant les étapes qui consistent à : (1) ajouter une solution de NaCl ou de chlorhydrate dans la dispersion et à (2) mélanger intimement le mélange qui en résulte pendant une période de temps suffisamment longue pour atténuer sensiblement la couleur jaunâtre des nanoparticules d'Ag dans la dispersion.

**4.** Procédé selon la revendication 1, 2 ou 3, dans lequel le prépolymère contenant de la silicone est au moins un élément choisi dans le groupe constitué par un prépolymère ayant au moins un groupe éthyléniquement insaturé, un prépolymère ayant deux groupes thiol ou plus, un prépolymère ayant au moins un groupe ène ; dans lequel le macromère contenant de la silicone est au moins un élément choisi dans le groupe constitué par un macromère ayant au moins un groupe éthyléniquement insaturé, un macromère ayant deux groupes thiol ou plus, un macromère ayant au moins un groupe ène ; dans lequel le monomère contenant de la silicone est au moins un élément choisi dans le groupe constitué par un monomère ayant un groupe éthyléniquement insaturé, un monomère ayant deux groupes thiol, un monomère ayant un groupe ène ; le groupe ène étant défini par l'une quelconque des formules (I) - (III)

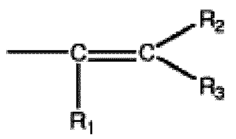

(I)

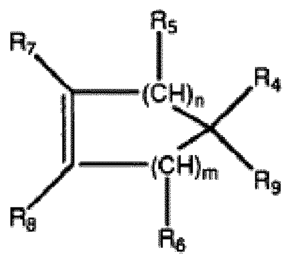

(II)

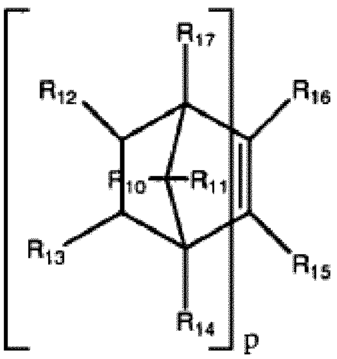

(III)

où $R_1$ est un hydrogène, ou un alkyle en $C_1$-$C_{10}$ ; $R_2$ et $R_3$, indépendamment l'un de l'autre, sont un hydrogène, un radical alcène en $C_1$-$C_{10}$ divalent, un alkyle en $C_1$-$C_{10}$, ou $-(R_{18})_a-(X_1)_b-R_{19}$ dans lequel $R_{18}$ est un radical alcène en $C_1$-$C_{10}$ divalent, $X_1$ est une liaison éther (-O-), une liaison uréthane (-N), une liaison urée, une liaison ester, une liaison amide, ou un carbonyle, $R_{19}$ est un hydrogène, une liaison simple, un groupe amino, un groupe carboxyle, un groupe hydroxyle, un groupe carbonyle, un groupe aminoalkyle en $C_1$-$C_{12}$, un groupe alkyl-en $C_1$-$C_{18}$ aminoalkyle, un groupe carboxyalkyle en $C_1$-$C_{18}$, un groupe hydroxyalkyle en $C_1$-$C_{18}$, un groupe alkyl-en $C_1$-$C_{18}$ alcoxy, un groupe aminoalcoxy en $C_1$-$C_{12}$, un groupe alkyl-en $C_1$-$C_{18}$ aminoalcoxy, un groupe carboxyalcoxy en $C_1$-$C_{18}$, ou un groupe hydroxyalcoxy en $C_1$-$C_{18}$, a et b sont, indépendamment l'un de l'autre, zéro ou 1, à condition que seulement l'un de $R_2$ et de $R_3$ soit un radical divalent ; $R_4$ - $R_9$ sont, indépendamment les uns des autres, un hydrogène, un radical alcène en $C_1$-$C_{10}$ divalent, un alkyle en $C_1$-$C_{10}$, ou $-(R_{18})_a-(X_1)_b-R_{19}$, éventuellement $R_4$ et $R_9$ sont liés par l'intermédiaire d'un radical alcène divalent de manière à former un cycle cyclique, à condition que l'un au moins de $R_4$- $R_9$ soit un radical divalent ; n et m sont, indépendamment l'un de l'autre, un nombre entier de 0 à 9, à condition que la somme de n et de m soit un nombre entier de 2 à 9 ; $R_{10}$ - $R_{17}$ sont, indépendamment les uns des autres, un hydrogène, un radical alcène en $C_1$-$C_{10}$ divalent, un alkyle en $C_1$-$C_{10}$, ou $-(R_{18})_a-(X_1)_b-R_{19}$ , p est un nombre entier de 1 à 3, à condition que seulement un ou deux des $R_{10}$ - $R_{17}$ soient des radicaux divalents.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4472327 A
- US 5358688 A
- US 5536861 A
- EP 0604369 A
- EP 0947856 A2
- US 5515117 A
- US 5213801 A
- US 5328954 A
- WO 2002062402 A
- US 20050013842 A1
- US 2007003603 A
- US 60869812 B
- US 4536554 A
- US 4983702 A
- US 5087392 A
- US 5656210 A
- US 6627124 B
- US 4312575 A
- US 4632844 A
- US 6451871 B
- US 6719929 B
- US 6793973 B
- US 6811805 B
- US 6896926 B
- US 5760100 A
- US 20010037001 A1
- US 6039913 A
- US 86981206 P
- EP 632329 A
- US 4444711 A, Schad
- US 4460534 A, Boehm
- US 5843346 A, Morrill
- US 5894002 A, Boneber
- US 09774942 B
- US 09775104 B
- US 60409950 B


### Non-patent literature cited in the description


- **CHALKLEY et al.** *Am. J. Ophthalmology,* 1966, vol. 61, 866-869
- *CHEMICAL ABSTRACTS,* 17096-07-0
- **WINTERTON et al.** The Cornea: Transactions of the World Congress on the Cornea 111. Raven Press, 1988, 273-280